(19) Europäisches Patentamt
European Patent Office
Office européen des brevets

(11) EP 2 022 493 A1

(12) # EUROPEAN PATENT APPLICATION

(43) Date of publication:
**11.02.2009 Bulletin 2009/07**

(51) Int Cl.:
***A61K 31/166*** *(2006.01)* ***A61P 33/00*** *(2006.01)*

(21) Application number: **07114034.7**

(22) Date of filing: **08.08.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Bayer CropScience AG**
**40789 Monheim (DE)**

(72) Inventors:
- **Fischer, Dr. Rüdiger**
  **50259 Pulheim (DE)**
- **Funke, Dr. Christian**
  **42799 Leichlingen (DE)**
- **Görgens, Ulrich**
  **40882 Ratingen (DE)**
- **Sirinyan, Dr. Kirkor**
  **51467 Bergisch Gladbach (DE)**
- **Turberg, Dr. Andreas**
  **42781 Haan (DE)**

(54) **Composition comprising at least one optically active phthalamide derivative for controlling animal parasites**

(57) The invention relates to a composition comprising at least one optically active phthalamide compound or a salt thereof for controlling animal parasites, veterinary pharmaceutical compositions comprising at least one optically active phthalamide of formula (I) for preventing infection with diseases transmitted through parasites, its use for the preparation of a veterinary pharmaceutical for controlling animal parasites, and a method for preventing infection with diseases transmitted through parasites.

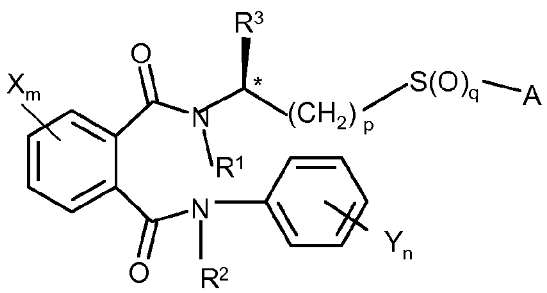

(I)

EP 2 022 493 A1

## Description

The invention relates to a composition comprising at least one optically active phthalamide compound or a salt thereof for controlling animal parasites, veterinary pharmaceutical compositions comprising at least one optically active phthalamide compound or a salt thereof for preventing infection with diseases transmitted through parasites, its use for the preparation of a veterinary pharmaceutical for controlling animal parasites, and a method for preventing infection with diseases transmitted through parasites.

The infestation of animals, domestic animals, companion animals and agricultural livestock alike, with parasites represents a problem. Often, the infested animals are infected with diseases transmitted through parasites, like e.g. lyme disease, a variety of sometimes fatal viral diseases or the immune system of the animals becomes weak due to the infestation, so that the animals become prone to other diseases, like e.g. bacterial infections. With the result that costly medicaments have to be administered, and, if the treated animals are agricultural livestock then the food safety is jeopardized. Additionally, the infestation of agricultural livestock with parasites is very often accompanied with a decrease in performance in terms of quality and quantity of produced meat, milk, egg, wool, or fur.

In particular, blood-sucking ectoparasites and ectoparasites causing myiasis are potential transmitters of a broad variety of pathogens besides all other secondary effects of the ectoparasitic infestation as there are blood-loss, irritation, inflammation, secondary bacterial infection, secondary parasiticidal infection (e.g. myiasis) and direct toxicosis (tick paralysis).

Although there are several compounds known to combat animal parasites there is still a need for new compounds. Especially in the field of livestock the treated ectoparasites are often present in high numbers. Together with repeated treatments necessary minimize damage to the host animals there is a steadily increasing risk that the ectoparasites are developing a resistance against the existing veterinary pharmaceuticals. There is also a need for veterinary pharmaceuticals which prevent the infestation of animals with parasites. And, moreover, for compounds which can prevent blood meal or lesions caused by ectoparasites and thus additionally can reduce the risk of transmitting vector-borne diseases to animals and humans.

From EP 1 006 107 A1 it is known that certain phthalamide compounds can be used as insecticides in the agricultural and horticultural field. Tohnishi et al. describe that Flubendiamide, a representative of said phthalamide compounds, is especially useful against lepidopterous pests including resistant strains (Tohnishi, M., et. al., J. Pestic. Sci., 30(4), 354-360 (2005)). EP 1 782 689 A2 describes that certain optically active phthalamide compounds are useful against agricultural and horticultural insect pests, especially against lepidopterous pests.

The inventors now surprisingly found that optically active phthalamide compounds or compositions comprising at least one optically active phthalamide compound exhibit excellent activity against animal parasites and thus can be used as veterinary pharmaceutical, especially for preventing infection with diseases which are transmitted through animal parasites.

Therefore, in a first embodiment, the invention relates to a pharmaceutical composition comprising at least one optically active phthalamide compound of formula (I), or a salt thereof

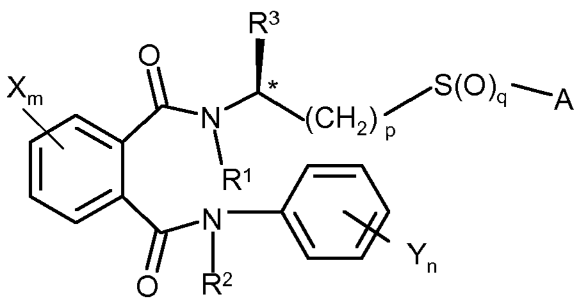

(I)

wherein

$R^1$ and $R^2$ independently of each other represents hydrogen, $(C_1-C_6)$-alkyl, $(C_1-C_6)$-alkyl which is substituted with at least one substituent selected from the group consisting of halogen, cyano, $(C_1-C_6)$-alkoxy, $(C_1-C_6)$-alkylthio, mono-$(C_1-C_6)$-alkylamino, and di-$(C_1-C_6)$-alkylamino in which the alkyl groups may be same or different, or $(C_1-C_6)$-alkoxycarbonyl;

$R^3$ represents $(C_1-C_6)$-alkyl;

A represents hydrogen, $(C_1-C_6)$-alkyl, halo$(C_1-C_6)$-alkyl, $(C_3-C_6)$-alkeny, halo$(C_3-C_6)$-alkenyl, $(C_3-C_6)$-alkynyl, halo $(C_3-C_6)$-alkynyl, $(C_1-C_6)$-alkoxy$(C_1-C_6)$-alkyl, halo$(C_1-C_6)$-alkoxy$(C_1-C_6)$alkyl, $(C_1-C_6)$-alkylthio$(C_1-C_6)$-alkyl, halo $(C_1-C_6)$-alkylthio$(C_1-C_6)$-alkyl, $(C_1-C_6)$-alkylsulfinyl$(C_1-C_6)$-alkyl, halo$(C_1-C_6)$alkylsulfinyl$(C_1-C_6)$-alkyl, $(C_1-C_6)$-alkylsulfonyl$(C_1-C_6)$-alkyl, halo$(C_1-C_6)$alkylsulfonyl$(C_1-C_6)$-alkyl, tri-$(C_1-C_6)$-alkylsilyl, $(C_1-C_6)$-alkylcarbonyl, mono-$(C_1-C_6)$-alkyl-carbamoyl, di-$(C_1-C_6)$-alkylcarbamoyl in which the alkyl groups may be same or different, phenoxy $(C_1-C_6)$-alkyl, substituted phenoxy $(C_1-C_6)$-alkyl having one or more substituents that may be same or different and are selected from
the group consisting of halogen, $(C_1-C_6)$-alkyl, halo$(C_1-C_6)$-alkyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo $(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di-(halo$(C_1-C_6)$-alkyl)amino in which the alkyl groups may be same or different, phenyl$(C_1-C_6)$-alkyl, or substituted phenyl$(C_1-C_6)$-alkyl wherein the ring is substituted with, one or more substituents that may be same or different and are selected from the group consisting of halogen, $(C_1-C_6)$-alkyl, halo$(C_1-C_6)$-alkyl, $(C_1-C_6)$-alkoxy, halo $(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di-(halo $(C_1-C_6)$-alkyl)amino in which the alkyl groups may be same or different;

p represents 0, 1, 2, 3 or 4;

q represents 0, 1, or 2;

X may be same or different and represents halogen, cyano, amino, nitro, $(C_1-C_6)$-alkyl, halo$(C_1-C_6)$-alkyl, $(C_2-C_6)$-alkenyl, halo$(C_2-C_6)$-alkenyl, $(C_2-C_6)$-alkynyl, halo$(C_2-C_6)$-alkynyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, $(C_1-C_6)$-alkylsulfonyloxy, halo$(C_1-C_6)$-alkylsulfonyl, mono-$(C_1-C_6)$-alkylamino, or di-$(C_1-C_6)$-alkylamino in which the alkyl groups may be same or different; and

m represents 0, 1, 2, 3 or 4; and

Y may be same or different and represents hydrogen, halogen, cyano, amino, nitro, $(C_1-C_6)$-alkyl, halo$(C_1-C_6)$-alkyl, $(C_2-C_6)$-alkenyl, halo$(C_2-C_6)$-alkenyl, $(C_2-C_6)$-alkynyl, halo$(C_2-C_6)$-alkynyl, $(C_3-C_6)$-cycloalkyl, halo$(C_3-C_6)$-cycloalkyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo $(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino, in which the alkyl groups may be same or different, tri-$(C_1-C_6)$-alkylsilyl in which the alkyl groups may be same or different, phenyl, substituted phenyl having one or more substituents that may be same or different and are selected from the group consisting of halogen, $(C_1-C_6)$-alkyl, halo$(C_1-C_6)$-alkyl, $(C_1-C_6)$-alkoxy group, halo $(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di(halo $(C_1-C_6)$-alkyl)amino in which the alkyl groups may be same or different, heterocyclic, or a substituted heterocyclic having one or more substituents that may be same or different and are selected from the group consisting of halogen, $(C_1-C_6)$-alkyl, halo$(C_1-C_6)$-alkyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di-(halo$(C_1-C_6)$-alkyl)amino in which the alkyl groups may be same or different; and

n represents 1, 2, 3, 4, or 5;

for preventing infection with diseases transmitted through parasites.

In a second embodiment, the pharmaceutical composition, comprising at least one optically active phthalamide compound of formula (I) as described in the first embodiment is a veterinary pharmaceutical composition.

In a third embodiment, the composition as described in the first or second embodiment comprises a compound of formula (I) wherein

$R^1$ and $R^2$ independently of each other represents hydrogen or $(C_1-C_6)$-alkyl;

$R^3$ represents methyl or ethyl;

A represents ($C_1$-$C_6$)-alkyl, ($C_3$-$C_6$)-alkenyl, ($C_3$-$C_6$)-alkynyl, ($C_1$-$C_6$)-alkoxy($C_1$-$C_6$)-alkyl, ($C_1$-$C_6$)-alkylthio ($C_1$-$C_6$)-alkyl, ($C_1$-$C_6$)-alkylsulfinyl($C_1$-$C_6$)-alkyl, ($C_1$-$C_6$)-alkylsulfonyl($C_1$-$C_6$)-alkyl, or a mono-($C_1$-$C_6$)-alkylcarbamoyl group;

p represents 0, 1, 2, 3 or 4;

q represents 0, 1, or 2;

X may be same or different and represents halogen, nitro, halo($C_1$-$C_6$)-alkyl, halo($C_1$-$C_6$)-alkoxy, ($C_1$-$C_6$)-alkylthio, ($C_1$-$C_6$)-alkylsulfinyl, ($C_1$-$C_6$)-alkylsulfonyl, ($C_1$-$C_6$)-alkylsulfonyloxy;

m represents 0, 1, or 2;

Y may be same or different and represents halogen, cyano, ($C_1$-$C_6$)-alkyl, halo($C_1$-$C_6$)-alkyl, ($C_2$-$C_6$)-alkenyl, halo ($C_2$-$C_6$)-alkenyl, ($C_2$-$C_6$)-alkynyl, halo($C_2$-$C_6$)-alkynyl, ($C_1$-$C_6$)-alkoxy, halo($C_1$-$C_6$)-alkoxy, ($C_1$-$C_6$)-alkylthio, halo ($C_1$-$C_6$)-alkylthio, ($C_1$-$C_6$)-alkylsulfinyl, halo($C_1$-$C_6$)-alkylsulfinyl, ($C_1$-$C_6$)-alkylsulfonyl, halo($C_1$-$C_6$)-alkylsulfonyl, mono-(halo($C_1$-$C_6$)-alkyl)amino, or di-(halo($C_1$-$C_6$)-alkyl)amino in which the alkyl groups may be same or different;

and

n represents 0, 1, 2, 3, or 4.

In a fourth embodiment the composition as described in the first or second embodiment comprises at least one compound of formula (I), wherein

$R^1$ and $R^2$ independently of each other represents hydrogen or ($C_1$-$C_6$)-alkyl;

$R^3$ represents methyl;

A represents ($C_1$-$C_6$)-alkyl, ($C_3$-$C_6$)-alkenyl, ($C_3$-$C_6$)-alkynyl, ($C_1$-$C_6$)-alkoxy($C_1$-$C_6$)-alkyl, ($C_1$-$C_6$)-alkylthio ($C_1$-$C_6$)-alkyl, ($C_1$-$C_6$)-alkylsulfinyl($C_1$-$C_6$)-alkyl, or ($C_1$-$C_6$)-alkylsulfonyl($C_1$-$C_6$)-alkyl;

p represents 1, or 2;

q represents 0, 1 or 2;

X may be same or different and represents halogen, nitro, halo($C_1$-$C_6$)-alkyl, or halo ($C_1$-$C_6$)-alkoxy, ($C_1$-$C_6$)-alkylthio, ($C_1$-$C_6$)-alkylsulfinyl, ($C_1$-$C_6$)-alkylsulfonyl, ($C_1$-$C_6$)-alkylsulfonyloxy;

m represents 1, or 2;

and

Y may be same or different and represents halogen, cyano, ($C_1$-$C_6$)-alkyl, halo($C_1$-$C_6$)-alkyl, ($C_2$-$C_6$)-alkenyl, halo ($C_2$-$C_6$)-alkenyl, ($C_2$-$C_6$)-alkynyl, halo($C_2$-$C_6$)-alkynyl, ($C_1$-$C_6$)-alkoxy, halo($C_1$-$C_6$)-alkoxy, ($C_1$-$C_6$)-alkylthio, halo ($C_1$-$C_6$)-alkylthio, ($C_1$-$C_6$)-alkylsulfinyl, halo($C_1$-$C_6$)-alkylsulfinyl, ($C_1$-$C_6$)-alkylsulfonyl, or halo($C_1$-$C_6$)-alkylsulfonyl, and

n represents 1, 2 or 3.

In a fifth embodiment, the composition as described in the first or second embodiment, comprises at least (S)-3-iodo-$N^1$-{2-methyl-4-[1,2,2,2-tetrafluoro-1-(trifluoromethyl)ethyl]phenyl}-$N^2$-(1-methyl-2-methylsulfonylethyl)phthalamide, and/or (S)-3-chloro-N'-{2-methyl-4-[1,2,2,2-tetrafluoro-1-(trifluoromethyl)ethyl]phenyl}-$N^2$-(1-methyl-2-methylsulfonylethyl)phthalamide, and/or (S)-3-bromo-$N^1$-{2-methyl-4-[1,2,2,2-tetrafluoro-1-(trifluoromethyl)ethyl]phenyl}-$N^2$-(1-methyl-2-methylsulfonylethyl)phthalamide, and/or (S)-3-iodo-$N^1$-{2-methyl-4-[1,2,2,2-tetrafluoro-1-(trifluoromethyl) ethyl]phenyl}-$N^2$-(1-methyl-2-methylsulfinylethyl) phthalamide, or their corresponding salts

In a sixth embodiment, the invention relates to the use of the optically active phthalamide compounds as described in any of the first to fifth embodiment for controlling parasites, preferably animal parasites, most preferred

ectoparasites.

In a seventh embodiment the invention relates to the use of a composition comprising at least one optically active phthalamide compound as described in any of the first to fifth embodiment for controlling parasites, preferably animal parasites, such as arthropods, more preferred ectoparasites, like ticks, mites, flies, lice and fleas, in particular, hard ticks, soft ticks, scab mites, harvest mites, stinging flies, licking flies, parasitic fly larvae, lice, hair lice, bird mites and bird lice.

In a eighths embodiment the invention relates to a method for preventing infection with diseases transmitted through parasites, characterized in that a composition comprising at least one optically active compound as described in any of the first to fifth embodiment is administered to the animal, preferably externally administered.

Moreover, it has been found that the compounds of the present invention provide excellent activity against animal parasites, particularly against arthropods attacking and/or infesting companion animals or agricultural livestock. Thus, the compounds and compositions of the invention can be used to control arthropods attacking and/or infesting companion animals and agricultural livestock.

The compounds according to the invention are known from EP 1 782 689 A2 and can be synthesized according to the methods described therein.

The composition according to the invention optionally comprises further active ingredients and/or auxiliary agents, such as for example developers, surfactants, emulsifiers, solvents, foam formers or anti-foaming agents and fillers.

Examples of further active ingredients which can be used in the present invention are insecticides, bactericides, acaricides, nematicides, fungicides. Examples of such active ingredients include organic phosphorous agents, carbonate agents, chemicals of the carboxylate type, chemicals of the chlorinated hydrocarbon type and materials produced from microorganisms.

Other examples of such active ingredients include, but are not limited to, Acetylcholinesterase (AChE) inhibitors, like carbamates, such as for example alanycarb, aldicarb, aldoxycarb, allyxycarb, aminocarb, bendiocarb, benfuracarb, bufencarb, butacarb, butocarboxim, butoxycarboxim, carbaryl, carbofuran, carbosulfan, cloethocarb, dimetilan, ethiofencarb, fenobucarb, fenothiocarb, formetanate, furathiocarb, isoprocarb, metam-sodium, methiocarb, methomyl, metolcarb, oxamyl, pirimicarb, promecarb, propoxur, thiodicarb, thiofanox, trimethacarb, XMC, xylylcarb, and triazamate; organophosphates, such as for example acephate, azamethiphos, azinphos (-methyl, -ethyl), aromophos-ethyl, aromfenvinfos (-methyl), autathiofos, cadusafos, carbophenothion, chlorethoxyfos, chlorfenvinphos, chlormephos, chlorpyrifos (-methyl/- ethyl), coumaphos, cyanofenphos, cyanophos, chlorfenvinphos, demeton-S-methyl, demeton-S-methylsulphone, dialifos, diazinone, dichlofenthione, dichlorvos/DDVP, dicrotophos, dimethoate, dimethylvinphos, dioxabenzofos, disulfoton, EPN, ethion, ethoprophos, etrimfos, famphur, fenamiphos, fenitrothion, fensulfothion, fenthion, flupyrazofos, fonofos, formothion, fosmethilan, fosthiazate, heptenophos, iodofenphos, iprobenfos, isazofos, isofenphos, isopropyl O-salicylate, isoxathion, malathion, mecarbam, methacrifos, methamidophos, methidathion, mevinphos, monocrotophos, naled, omethoate, oxydemeton-methyl, parathion (-methyl/-ethyl), phenthoate, phorate, phosalone, phosmet, phosphamidone, phosphocarb, Phoxim, pirimiphos (-methyl/-ethyl), profenofos, propaphos, propetamphos, prothiofos, prothoate, pyraclofos, pyridaphenthion, pyridathion, quinalphos, sebufos, sulfotep, sulprofos, tebupirimfos, temephos, terbufos, tetrachlorvinphos, thiometon, triazophos, triclorfon, vamidothion.

Sodium channel modulators / voltage-dependent sodium channel blockers like pyrethroids, such as for example acrinathrin, allethrin (d-cis-trans, d-trans), beta-cyfluthrin, bifenthrin, bioallethrin, bioallethrin-S-cyclopentyl-isomer, bioethanomethrin, biopermethrin, bioresmethrin, chlovaporthrin, cis-cypermethrin, cis-resmethrin, cis-permethrin, clocythrin, cycloprothrin, cyfluthrin, cyhalothrin, cypermethrin (alpha-, beta-, theta-, zeta), cyphenothrin, deltamethrin, empenthrin (1R-isomer), esfenvalerate, etofenprox, fenfluthrin, fenpropathrin, fenpyrithrin, fenvalerate, flubrocythrinate, flucythrinate, flufenprox, flumethrin, fluvalinate, fubfenprox, gamma-cyhalothrin, imiprothrin, kadethrin, lambda-cyhalothrin, metofluthrin, permethrin (cis-, trans-), phenothrin (1R-trans isomer), prallethrin, profluthrin, protrifenbute, pyresmethrin, resmethrin, RU 15525, silafluofen, taufluvalinate, tefluthrin, terallethrin, tetramethrin (-1R- isomer), tralomethrin, transfluthrin, ZXI 8901, pyrethrins (pyrethrum);

DDT; oxadiazines, such as for example indoxacarb.

Acetylcholine receptor agonists/antagonists, like chloronicotinyls, such as for example acetamiprid, clothianidin, dinotefuran, imidacloprid, nitenpyram, nithiazine, thiacloprid, thiamethoxam, nicotine, bensultap, cartap.

Acetylcholine receptor modulators, like Spinosynes, such as for example spinosad.

GABA controlled chloride channel antagonists, like Organochlorinee, such as for example camphechlor, chlordane, endosulfan, gamma-HCH, HCH, heptachlor, lindane, methoxychlor; Fiproles, such as for example acetoprole, ethiprole, fipronil, pyrafluprole, pyriprole, and vaniliprole.

Chloride channel activators, like Mectins, such as for example avermectin, emamectin, emamectin benzoate, ivermectin, milbemycin, latidectin, lepimectin, selamectin, doramectin, eprinomectin, and moxidectin.

Juvenile hormone mimetics, like for example diofenolan, epofenonane, fenoxycarb, hydroprene, kinoprene, methoprene, pyriproxifen, and triprene.

Latrophilin receptor agonists, like depsipeptides, referably cyclic depsipetides, in particular 24-membered cyclic depsipeptides, for example emodepside.
Ecdysone agonists/disruptors, like diacylhydrazines, such as for example chromafenozide, halofenozide, methoxyfenozide, tebufenozide.
Inhibitors of chitin biosynthesis, like Benzoylureas, such as for example bistrifluron, chlofluazuron, diflubenzuron, fluazuron, flucycloxuron, flufenoxuron, hexaflumuron, lufenuron, novaluron, noviflumuron, penfluron, teflubenzuron, triflumuron; buprofezin; cyromazine.
Inhibitors of oxidative phosphorylation, ATP disruptors such as diafenthiuron; organotin compounds, such as for example azocyclotin, cyhexatin, fenbutatin-oxide.
Decouplers of oxidative phosphorylation by interruption of H-proton gradients like pyrrole, such as for example chlorfenapyr; dinitrophenols, such as for example binapacyrl, dinobuton, dinocap, DNOC.
Site I electron transport inhibitors, like METI's, such as for example fenazaquin, fenpyroximate, pyrimidifen, pyridaben, tebufenpyrad, tolfenpyrad; hydramethylnon; dicofol.
Site II electron transport inhibitors, like rotenones.
Site III electron transport inhibitors, like acequinocyl, fluacrypyrim.
Microbial disruptors of insect intestinal membrane such Bacillus thuringiensis strains.
Inhibitors of fat synthesis, like tetronic acids, such as for example spirodiclofen, spiromesifen; tetramic acids, such as for example spirotetramat (CAS-Reg.-No.: 203313-25-1) and 3-(2,5-dimethylphenyl)-8-methoxy-2-oxo-1-azaspiro[4.5]dec-3-en-4-yl ethyl carbonate (alias: carbonic acid, 3-(2,5-dimethylphenyl)-8-methoxy-2-oxo-1-azaspiro[4.5] dec-3-en-4-yl ethyl ester, CAS-Reg.-No.: 382608-10-8); carboxamides, such as for example flonicamid.
Octopaminergic agonists, such as for example amitraz.
Inhibitor of magnesium-stimulated ATPase, like propargite benzoic acid dicarboxamides, such as for example flubendiamide; Nereistoxin analogous, such as for example thiocyclam hydrogen oxalate, thiosultap-sodium.
Biologicals, hormones or pheromones like azadirachtin, Bacillus spec., Beauveria spec., codlemone, Metarrhizium spec., Paecilomyces spec., thuringiensin, Verticillium spec.
Active ingredients with unknown or non-specific mode of action, like fumigants, such as for example aluminium phosphide, methyl bromide, sulphuryl fluoride; feeding inhibitors, such as for example cryolite, flonicamid, pymetrozine; mite growth inhibitors, such as for example clofentezine, etoxazole, hexythiazox; amidoflumet, benclothiaz, benzoximate, bifenazate, bromopropylate, buprofezin, quinomethionate, chlordimeform, chlorobenzilate, chloropicrin, clothiazoben, cycloprene, cyflumetofen, dicyclanil, fenoxacrim, fentrifanil, flubenzimine, flufenerim, flutenzin, gossyplure, hydramethylnone, japonilure, metoxadiazone, petroleum, piperonyl butoxide, potassium oleate, pyridalyl, sulfluramid, tetradifon, tetrasul, triarathene, and verbutin.
Examples for parasites against which the compounds or compositions of the invention can be used include, but are not limited to, the above mentioned and, endoparasites, like for example helminthes such as Acanthocephala, Ascariasis, Cestoda, Clonorchis sinensis, Dracunculiasis, Enterobius vermicularis, Filariasis, Hookworm, Loa loa, Onchocerciasis, Schistosomiasis, Strongyloides stercoralis, Toxocara canis, Trichinella, Whipworm, arthropods like for example Gastrophilus spp, Stomoxys spp, Trichodectes spp, Rhodnius spp, Ctenocephalides canis, Cimx lecturius, Ctenocephalides felis and Lucilia cuprina, and, from the order of Acari they include for example Acarina Ornithodoros spp., Ixodes spp. and Boophilus spp..
Further examples of parasites include but are not limited to parasites
from the order of Anoplurida, such as Haematopinus spp., Linognathus spp., Pediculus spp., Phtirus spp., Solenopotes spp., Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
from the order of the Mallophagida and the suborders Amblycerina and Ischnocerina, such as Trimenopon spp., Menopon spp., Trinoton spp., Bovicola spp., Werneckiella spp., Lepikentron spp., Damalina spp., Trichodectes spp., Felicola spp., Bovicola bovis, Bovicola ovis, Bovicola limbata, Damalina bovis, Trichodectes canis, Felicola subrostratus, Bovicola caprae, Lepikentron ovis, Werneckiella equi;
from the order of the Diptera and the suborders Nematocerina and Brachycerina, such as Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp., Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;

from the order of the Siphonapterida, such as Pulex spp., Ctenocephalides spp., Tunga spp., Xenopsylla spp., Ceratophyllus spp., Ctenocephalides canis, Ctenocephalides felis, Pulex irritans, Tunga penetrans, Xenopsylla cheopis;

from the order of the Heteropterida, such as Cimex spp., Triatoma spp., Rhodnius spp., Panstrongylus spp.;

from the order of the Blattarida, such as Blatta orientalis, Periplaneta americana, Blattela germanica, Supella spp., Suppella longipalpa;

from the subclass of the Acari (Acarina) and the orders of the Meta- and Mesostigmata, such as Argas spp., Ornithodorus spp., Otobius spp., Ixodes spp., Amblyomma spp., Rhipicephalus (Boophilus) spp Dermacentor spp., Haemophysalis spp., Hyalomma spp., Dermanyssus spp., the original genus of multi host ticks, namely Rhipicephalus spp., Ornithonyssus spp., Pneumonyssus spp., Raillietia spp., Pneumonyssus spp., Sternostoma spp., Varroa spp., Acarapis spp., Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;

from the order of the Actinedida (Prostigmata) and Acaridida (Astigmata), such as for example Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp., Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae or Sarcoptes caprae, Sarcoptes equi, Sarcoptes suis, Psoroptes ovis, Psoroptes cuniculi, Psoroptes equi, Chorioptes bovis, Psoergates ovis, Pneumonyssoidic Mange, Pneumonyssoides caninum, Acarapis woodi.

Diseases transmitted through parasites, particularly ectoparasites are for example bacterial, viral, rickettsial and protozoal vector-borne diseases.

Examples of viral diseases transmitted through arboviruses, i.e. arthropod borne viruses, are Crimean-Congo Hemorhagic Fever (CCHF), Febrile illness, Papataci fever, Encephalitis, Meningitis, which are caused by Bunyaviridae such as Bunyavirus, Nairovirus or Phlebovirus; Bluetongue, meningoencephalits, Febrile illness, hemorhagic fever, which are caused by Reoviridae, such as Orbivirus, Colitivirus; Febrile illness, rash, enzephalitis, polyarthritis, lymphadenitis, which are caused by Togaviridae, such as Sindbisvirus, Chikungunya Virus; tick-borne meningoencephalitis, Dengue hemorhagic fever, encephalitis, Febrile illness, Yellow fever, which are caused by Flaviviridae, such as Flavivirus ( including diverse sub-groups).

Examples of bacterial diseases transmitted through parasites are Rickettsiosis, such as Rocky Mountain spotted fever, tick typhus caused by infection through Rickettsia ssp; Tularemia caused by infection through Francisella tularensis; Borreliosis or Spirochaetosis, such as Lyme disease, or relapsing fever, caused by infection through Borrelia ssp.; Ehrllichiosis caused by infection through Ehrlichia ssp.; Plague, caused by infection through Yersinia ssp..

Examples of protozoal or rickettsial borne diseases are Babesiosis, such as texas fever, red water disease, Q-fever caused by infection through Babesia ssp.; Theileriosis, such as east coast fever, Mediterranean coast fever, caused by infection through Theileria ssp.; Nagana disease, Sleeping sickness caused by infection through Trypanosoma ssp., Anaplasmosis caused by infection through Anaplasma ssp.; Malaria caused by infection through Plasmodium ssp.; Leishmaniasis caused by infection through Leishmania ssp..

The diseases transmitted through parasites are numerous and are not only limited to the above mentioned. Further diseases caused by animal parasites, in particular ectoparasites, are Myiasis caused by parasites like e.g. Lucilia ssp.; Scabies caused by parasites like e.g. Sarcoptes ssp., Psoroptes ssp., Demodex ssp.; Pediculosis caused by parasites like Mallophaga (biting lice) Bovicola ssp., and Anoplura (sucking lice) Haematopinus ssp.; Flea allergic dermatitis caused by parasites like Ctenocephalides ssp., Pulex ssp.; Hypodermosis caused by parasites like e.g. Hypoderma ssp., Dermatobia ssp.; Ixodidiosis caused by parasites like e.g. Ixodes ssp., Rhipcephalus ssp., Hyalomma ssp., Amblyomma ssp..

According to the invention, the term "animals" include all kind of animals among that also humans, domestic animals, like conventional pet animals, such as for example dogs, cats, cage birds, aquarium fish, less conventional pet animals, such as ferrets, reptiles and exotic birds, all kind of experimental animals, such as rodents like for example, rats and mice, and hamsters and guinea pigs, and agricultural livestock.

Examples for agricultural livestock are cattle, sheep, goats, horses, pigs, donkeys, camels, buffaloes, rabbits, poultries, like chickens, turkeys, ducks, geese, honeybee, and fur animals such as mink.

By controlling animal parasites it is understood to combat the parasites or to prevent infestation through parasites. By combating animal parasites it is understood to reduce the absolute number of parasites on or in the host animal.

The compounds or composition according to the invention can be administered in a known manner and in an appropriate preparation form. Preference is given to enteral, parenteral, or external administration.

In general, the invention may be carried out in a manner fit to the form of application or administering. Suitable forms include aerosols, unpressurized sprays, for example pump sprays and atomizer sprays, automatic misting devices, foggers, foams, gels, vaporizer products with vaporizer platelets made of cellulose or polymer, liquid vaporizers, gel and membrane vaporizers, propeller-driven vaporizers, vaporization systems which do not consume energy (passive vaporization systems).

Moreover, the compounds and compositions according to the invention can be applied by way of intramuscular, subcutaneous, intravenous, intraperitoneal injections, implants, or nasal application; by dermal application in the form of, for example, bathing or dipping, spraying, pouring-on and spotting-on, washing, dusting, and with the aid of active-compound-comprising shaped articles such as collars, ear tags, tail tags, limb bands, halters, marking devices and the like. The preparation forms for administering the compounds and compositions according to the invention enterally can be tablets, capsules, drinks, drenches, granules, pastes, boluses, feed-through method, and suppositories.

In particular, the compounds and compositions according to the invention can be formulated into usual preparation forms. For the various ways of administration, examples of the preparation forms include solutions, emulsions, wettable powders, dry flowables, suspensions, dusts, foams, pastes, tablets, granules, aerosols, active compound infiltrated-natural and synthetic products, microcapsules, preparations with a combustor (for example, fumigating and smoking cartridges, cans and coils), ULV (cold mists and warm mists). Preference is given to powders, emulsions, flowables, homogeneous solutions, emulsion concentrate formulations, WP and suspensions, or suspension concentrate formulations. Particularly preferred are methods of application, like pourons, spot-ons, sprays, ear-tags and dips with the formulations mentioned herein.

Each of these formulations may be prepared by a known manner per se. Usually the compound or composition according to the invention is mixed with developers, such as liquid diluents or carriers, liquid gas diluents or carriers, solid diluents or carriers, and optionally with surfactants, such as anionic, kationic and non-ionic surfactants, such as dioctyl sodium sulfosuccinate and/or dispersants. Additionally to said developers and the optionally present surfactant and/or dispersant other auxiliary ingredients, like emulsifiers, foam former or anti-foaming agents, such as simethicone, preservatives, binders and/or colorants can be present in the formulation. The formulation of the active compound or composition preferably comprises a developer, an emulsifiers and/or dispersants and/or foam formers.

Examples of liquid diluents or carriers include, but are not limited to, aromatic hydrocarbons, such as xylene, toluene and alkylnaphthalene, chlorinated aromatic or aliphatic hydrocarbons, such as chlorobenzenes, ethylene chlorides and methylene chlorides, aliphatic hydrocarbons, such as cyclohexane, paraffins, such as mineral oil fractions, alcohols, such as for example, benzyl alcohol, isopropanol, ethanol, butanol, glycol and ethers and esters thereof, ketones, such as acetone, methyl ethyl ketone, methyl isobutyl ketone and cyclohexanone, strong polar solvents, such as dimethylformamide and dimethylsulfoxide, cyclic carbonates, such as ethylene carbonate, propylene carbonate, pyrrolidones, such as N-octylpyrrolidone, N- methylpyrrolidone, ethers, such as diethylene glycol monomethylether and diethylene glycol monopropylether, lactones, such as butyrolacton, and water.

Examples of liquid gas diluents or carriers include, but are not limited to, those which are in a gaseous state at room pressure and liquid under increased pressure, like for example aerosols propellants, such as fron, propane, nitrogen gas, carbon dioxide, and halogenated hydrocarbons.

Examples of solid diluents or carrier include, but are not limited to, ground natural minerals, such as kaolins, clays, talc, chalk, quartz, attapulgite, montmorillonite or diatomaceous earth, and ground synthetic minerals, such as highly dispersed silicic acid, alumina and silicate.

Examples of solid carriers for granules include, but are not limited to, crushed and fractionated rocks, like for example, calcite, marble, pumice, sepiolite and dolomite, synthetic granules of inorganic or organic powders, organic materials, like for example, sawdust, coconut shells, maize cobs and tobacco stalks.

Examples of emulsifiers and/or foam formers include, but are not limited to, nonionic and anionic emulsifiers, like for example, polyoxyethylene fatty acid esters, polyoxyethylene fatty acid alcohol ethers, such as for example, alkylaryl polyglycol ether, alkyl sulfonates, alkyl sulfates and aryl sulfonates and albumin hydrolysates.

Examples of active-compounds comprising shaped articles, such as for example collars include but are not limited o poly vinyl chloride, polyamide, polyamide 6, polyamide-6,6, polyolefines such as high densitiy polyethylene (HDPE), polyethylene, polypropylene and ethylene propylene diene monomer (EPDM).

Examples of dispersants include, but are not limited to, lignin sulfite waste liquor and methylcellulose.

Binders are used in preparations, like for example, powders, granules and emulsifiable concentrates. Examples of binders include, but are not limited to, starches, sugars, cellulose, or modified cellulose such as carboxymethylcellulose, hydroxypropyl cellulose, lactose, or sugar alcohols like xylitol, sorbitol or maltitol., natural or synthetic polymers, such as, gum arabicum, xanthane, polyvinyl alcohol and polyvinyl acetate.

Examples of the colorant include, but are not limited to, inorganic pigments, such as iron oxide, titanium oxide and Prussian blue, organic colorants such as Alizarin colorants, azo colorants or metal phthalocyanine colorants, and further, trace nutrients such as salts of iron, manganese, boron, copper, cobalt, molybdenum or zinc.

The formulation may contain the compound according to the invention from 0.1 to 95% by weight of the total preparation, preferably from 0.5 to 90% by weight, most preferred from 0.8 to 70 % by weight.

Representative compounds according to the invention are shown in the following table 1.

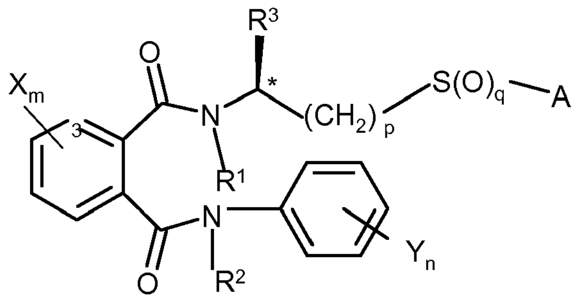

(I)

Table 1

| No. | $Y_n$ | $R^2$ | $X_m$ | $R^1$ | $R^{3*}$ | A | p | q | logP | melting point [°C] |
|---|---|---|---|---|---|---|---|---|---|---|
| 1 | 2-$CH_3$ 4-$CF(CF_3)CF_3$ | H | 3-1 | H | $CH_3$ | $CH_3$ | 1 | 2 | 3.18[a] 3.2[c] | 149-152 |
| 2 | 2-$CH_3$ 4-$CF(CF_3)CF_3$ | H | 3-$NO_2$ | H | $CH_3$ | $CH_3$ | 1 | 2 | | |
| 3 | 2-$CH_3$ 4-$CF(CF_3)CF_3$ | H | 3-$OSO_2CH_3$ | H | $CH_3$ | $CH_3$ | 1 | 2 | | |
| 4 | 2-$CH_3$ 4-$CF(CF_3)CF_3$ | H | 3-$SO_2CH_3$ | H | $CH_3$ | $CH_3$ | 1 | 2 | | |
| 5 | 2-$CH_3$ 4-$CF(CF_3)CF_3$ | H | 3-$SO_2CF_3$ | H | $CH_3$ | $CH_3$ | 1 | 2 | | |
| 6 | 2-$CH_3$ 4-$CF(CF_3)CF_3$ | H | 3-$CF_3$ | H | $CH_3$ | $CH_3$ | 1 | 2 | | |
| 7 | 2-$CH_3$ 4-(p-$CF_3$-phenyl) | H | 3-1 | H | $CH_3$ | $CH_3$ | 1 | 2 | | |
| 8 | 2-$CH_3$ 4-(p-$CF_3$-phenyl) | H | 3-Cl | H | $CH_3$ | $CH_3$ | 1 | 2 | | |
| 9 | 2-$CH_3$ 4-(p-$CF_3$-phenyl) | H | 3-Br | H | $CH_3$ | $CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 10 | 2-$CH_3$ 4-(p-$CF_3$-phenyl) | H | 3-$NO_2$ | H | $CH_3$ | $CH_3$ | 1 | 2 | | |
| 11 | 2-$CH_3$ 4-(p-$CF_3$-phenyl) | H | 3-$OSO_2CH_3$ | H | $CH_3$ | $CH_3$ | 1 | 2 | | |
| 12 | 2-$CH_3$ 4-(p-$CF_3$-phenyl) | H | 3-$SO_2CH_3$ | H | $CH_3$ | $CH_3$ | 1 | 2 | | |
| 13 | 2-$CH_3$ 4-(p-$CF_3$-phenyl) | H | 3-$SO_2CF_3$ | H | $CH_3$ | $CH_3$ | 1 | 2 | | |
| 14 | 2-$CH_3$ 4-(p-$CF_3$-phenyl) | H | 3-$CF_3$ | H | $CH_3$ | $CH_3$ | 1 | 2 | | |
| 15 | 2-$CH_3$ 4-(3-$CF_3$-pyrazole-1-yl) | H | 3-1 | H | $CH_3$ | $CH_3$ | 1 | 2 | | |
| 16 | 2-$CH_3$ 4-(3-$CF_3$-pyrazole-1-yl) | H | 3-Cl | H | $CH_3$ | $CH_3$ | 1 | 2 | | |
| 17 | 2-$CH_3$ 4-(3-$CF_3$-pyrazole-1-yl) | H | 3-Br | H | $CH_3$ | $CH_3$ | 1 | 2 | | |
| 18 | 2-$CH_3$ 4-(3-$CF_3$-pyrazole-1-yl) | H | 3-$NO_2$ | H | $CH_3$ | $CH_3$ | 1 | 2 | | |
| 19 | 2-$CH_3$ 4-(3-$CF_3$-pyrazole-1-yl) | H | 3-$OSO_2CH_3$ | H | $CH_3$ | $CH_3$ | 1 | 2 | | |
| 20 | 2-$CH_3$ 4-(3-$CF_3$-pyrazole-1-yl) | H | 3-$SO_2CH_3$ | H | $CH_3$ | $CH_3$ | 1 | 2 | | |
| 21 | 2-$CH_3$ 4-(3-$CF_3$-pyrazole-1-yl) | H | 3-$SO_2CF_3$ | H | $CH_3$ | $CH_3$ | 1 | 2 | | |
| 22 | 2-$CH_3$ 4-(3-$CF_3$-pyrazole-1-yl) | H | 3-$CF_3$ | H | $CH_3$ | $CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 23 | 2-$CH_3$ 4-$CH(CF_3)CF_3$ | H | 3-1 | H | $CH_3$ | $CH_2CH_3$ | 1 | 2 | | |
| 24 | 2-$CH_3$ 4-$CH(CF_3)CF_3$ | H | 3-1 | H | $CH_3$ | $CH_3$ | 1 | 0 | | |
| 25 | 2-$CH_3$ 4-$CH(CF_3)CF_3$ | H | 3-$NO_2$ | H | $CH_3$ | $CH_3$ | 1 | 0 | | |
| 26 | 2-$CH_3$ 4-$CH(CF_3)CF_3$ | H | 3-Cl | H | $CH_3$ | $CH_3$ | 1 | 0 | | |
| 27 | 2-$CH_3$ 4-$CH(CF_3)CF_3$ | H | 3-Br | H | $CH_3$ | $CH_3$ | 1 | 0 | | |
| 28 | 2-$CH_3$ 4-$CH(CF_3)CF_3$ | H | 3-$OSO_2CH_3$ | H | $CH_3$ | CH3 | 1 | 0 | | |
| 29 | 2-$CH_3$ 4-$CH(CF_3)CF_3$ | H | 3-$SO_2CH_3$ | H | $CH_3$ | $CH_3$ | 1 | 0 | | |
| 30 | 2-$CH_3$ 4-$CH(CF_3)CF_3$ | H | 3-$SO_2CF_3$ | H | $CH_3$ | $CH_3$ | 1 | 0 | | |
| 31 | 2-$CH_3$ 4-$CH(CF_3)CF_3$ | H | 3-$CF_3$ | H | $CH_3$ | $CH_3$ | 1 | 0 | | |
| 32 | 2-$CH_3$ 4-(p-$CF_3$-phenyl) | H | 3-1 | H | $CH_3$ | $CH_3$ | 1 | 0 | | |
| 33 | 2-$CH_3$ 4-(p-$CF_3$-phenyl) | H | 3-Cl | H | $CH_3$ | $CH_3$ | 1 | 0 | | |
| 34 | 2-$CH_3$ 4-(p-$CF_3$-phenyl) | H | 3-Br | H | $CH_3$ | $CH_3$ | 1 | 0 | | |
| 35 | 2-$CH_3$ 4-(p-$CF_3$-phenyl) | H | 3-$NO_2$ | H | $CH_3$ | $CH_3$ | 1 | 0 | | |
| 36 | 2-$CH_3$ 4-(p-$CF_3$-phenyl) | H | 3-$OSO_2CH_3$ | H | $CH_3$ | $CH_3$ | 1 | 0 | | |
| 37 | 2-$CH_3$ 4-(p-$CF_3$-phenyl) | H | 3-$SO_2CH_3$ | H | $CH_3$ | $CH_3$ | 1 | 0 | | |
| 38 | 2-$CH_3$ 4-(p-$CF_3$-phenyl) | H | 3-$SO_2CF_3$ | H | $CH_3$ | $CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 39 | 2-$CH_3$ 4-(p-$CF_3$-phenyl) | H | 3-$CF_3$ | H | $CH_3$ | $CH_3$ | 1 | 0 | | |
| 40 | 2-$CH_3$ 4-(3-$CF_3$-pyrazole-1-yl) | H | 3-1 | H | $CH_3$ | $CH_3$ | 1 | 0 | | |
| 41 | 2-$CH_3$ 4-(3-$CF_3$-pyrazole-1-yl) | H | 3-Cl | H | $CH_3$ | $CH_3$ | 1 | 0 | | |
| 42 | 2-$CH_3$ 4-(3-$CF_3$-pyrazole-1-yl) | H | 3-Br | H | $CH_3$ | $CH_3$ | 1 | 0 | | |
| 43 | 2-$CH_3$ 4-(3-$CF_3$-pyrazole-1-yl) | H | 3-$NO_2$ | H | $CH_3$ | $CH_3$ | 1 | 0 | | |
| 44 | 2-$CH_3$ 4-(3-$CF_3$-pyrazole-1-yl) | H | 3-$OSO_2CH_3$ | H | $CH_3$ | $CH_3$ | 1 | 0 | | |
| 45 | 2-$CH_3$ 4-(3-$CF_3$-pyrazole-1-yl) | H | 3-$SO_2CH_3$ | H | $CH_3$ | $CH_3$ | 1 | 0 | | |
| 46 | 2-$CH_3$ 4-(3-$CF_3$-pyrazole-1-yl) | H | 3-$SO_2CF_3$ | H | $CH_3$ | $CH_3$ | 1 | 0 | | |
| 47 | 2-$CH_3$ 4-(3-$CF_3$-pyrazole-1-yl) | H | 3-$CF_3$ | H | $CH_3$ | $CH_3$ | 1 | 0 | | |
| 48 | 2-$CH_3$ 4-$CH(CF_3)CF_3$ | H | 3-1 | H | $CH_3$ | $CH_3$ | 1 | 2 | | |
| 49 | 2-$CH_3$ 4-$CH(CF_3)CF_3$ | H | 3-Cl | H | $CH_3$ | $CH_3$ | 1 | 2 | | |
| 50 | 2-$CH_3$ 4-$CH(CF_3)CF_3$ | H | 3-Br | H | $CH_3$ | $CH_3$ | 1 | 2 | | |
| 51 | 2-$CH_3$ 4-$CH(CF_3)CF_3$ | H | 3-$NO_2$ | H | $CH_3$ | $CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 52 | 2-$CH_3$ 4-$CH(CF_3)CF_3$ | H | 3-$OSO_2CH_3$ | H | $CH_3$ | $CH_3$ | 1 | 2 | | |
| 53 | 2-$CH_3$ 4-$CH(CF_3)CF_3$ | H | 3-$SO_2CH_3$ | H | $CH_3$ | $CH_3$ | 1 | 2 | | |
| 54 | 2-$CH_3$ 4-$CH(CF_3)CF_3$ | H | 3-$SO_2CF_3$ | H | $CH_3$ | $CH_3$ | 1 | 2 | | |
| 55 | 2-$CH_3$ 4-$CH(CF_3)CF_3$ | H | 3-$CF_3$ | H | $CH_3$ | $CH_3$ | 1 | 2 | | |
| 56 | 2-$CH_3$ 4-$CH(CF_3)CF_3$ | H | 3-1 | H | $CH_3$ | $CH_3$ | 1 | 1 | | |
| 57 | 2-$CH_3$ 4-$CH(CF_3)CF_3$ | H | 3-Cl | H | $CH_3$ | $CH_3$ | 1 | 1 | | |
| 58 | 2-$CH_3$ 4-$CH(CF_3)CF_3$ | H | 3-Br | H | $CH_3$ | $CH_3$ | 1 | 1 | | |
| 59 | 2-$CH_3$ 4-$CH(CF_3)CF_3$ | H | 3-$NO_2$ | H | $CH_3$ | $CH_3$ | 1 | 1 | | |
| 60 | 2-$CH_3$ 4-$CH(CF_3)CF_3$ | H | 3-$OSO_2CH_3$ | H | $CH_3$ | $CH_3$ | 1 | 1 | | |
| 61 | 2-$CH_3$ 4-$CH(CF_3)CF_3$ | H | 3-$SO_2CH_3$ | H | $CH_3$ | $CH_3$ | 1 | 1 | | |
| 62 | 2-$CH_3$ 4-$CH(CF_3)CF_3$ | H | 3-$SO_2CF_3$ | H | $CH_3$ | $CH_3$ | 1 | 1 | | |
| 63 | 2-$CH_3$ 4-$CH(CF_3)CF_3$ | H | 3-$CF_3$ | H | $CH_3$ | $CH_3$ | 1 | 1 | | |
| 64 | 2-$CH_3$ 4-(p-$CF_3$-phenyl) | H | 3-1 | H | $CH_3$ | $CH_3$ | 1 | 1 | | |
| 65 | 2-$CH_3$ 4-(p-$CF_3$-phenyl) | H | 3-Br | H | $CH_3$ | $CH_3$ | 1 | 1 | | |
| 66 | 2-$CH_3$ 4-(p-$CF_3$-phenyl) | H | 3-Cl | H | $CH_3$ | $CH_3$ | 1 | 1 | | |
| 67 | 2-$CH_3$ 4-(p-$CF_3$-phenyl) | H | 3-$NO_2$ | H | $CH_3$ | $CH_3$ | 1 | 1 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 68 | 2-$CH_3$ 4-(p-$CF_3$-phenyl) | H | 3-$OSO_2CH_3$ | H | $CH_3$ | $CH_3$ | 1 | 1 | | |
| 69 | 2-$CH_3$ 4-(p-$CF_3$-phenyl) | H | 3-$SO_2CH_3$ | H | $CH_3$ | $CH_3$ | 1 | 1 | | |
| 70 | 2-$CH_3$ 4-(p-$CF_3$-phenyl) | H | 3-$SO_2CF_3$ | H | $CH_3$ | $CH_3$ | 1 | 1 | | |
| 71 | 2-$CH_3$ 4-(p-$CF_3$-phenyl) | H | 3-$CF_3$ | H | $CH_3$ | $CH_3$ | 1 | 1 | | |
| 72 | 2-$CH_3$ 4-(3-$CF_3$-pyrazole-1-yl) | H | 3-1 | H | $CH_3$ | $CH_3$ | 1 | 1 | | |
| 73 | 2-$CH_3$ 4-(3-$CF_3$-pyrazole-1-yl) | H | 3-Cl | H | $CH_3$ | $CH_3$ | 1 | 1 | | |
| 74 | 2-$CH_3$ 4-(3-$CF_3$-pyrazole-1-yl) | H | 3-Br | H | $CH_3$ | $CH_3$ | 1 | 1 | | |
| 75 | 2-$CH_3$ 4-(3-$CF_3$-pyrazole-1-yl) | H | 3-$NO_2$ | H | $CH_3$ | $CH_3$ | 1 | 1 | | |
| 76 | 2-$CH_3$ 4-(3-$CF_3$-pyrazole-1-yl) | H | 3-$OSO_2CH_3$ | H | $CH_3$ | $CH_3$ | 1 | 1 | | |
| 77 | 2-$CH_3$ 4-(3-$CF_3$-pyrazole-1-yl) | H | 3-$SO_2CH_3$ | H | $CH_3$ | $CH_3$ | 1 | 1 | | |
| 78 | 2-$CH_3$ 4-(3-$CF_3$-pyrazole-1-yl) | H | 3-$SO_2CF_3$ | H | $CH_3$ | $CH_3$ | 1 | 1 | | |
| 79 | 2-$CH_3$ 4-(3-$CF_3$-pyrazole-1-yl) | H | 3-$CF_3$ | H | $CH_3$ | $CH_3$ | 1 | 1 | | |
| 80 | 2-$CH_3$ 4-$CF(CF_3)CF_3$ | H | 3-Cl | H | $CH_3$ | $CH_3$ | 1 | 2 | | 168-170 |
| 81 | 2-$CH_3$ 4-$CF(CF_3)CF_3$ | H | 3-1 | H | $CH_3$ | $CH_2CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 82 | 2-$CH_3$ 4-$CF(CF_3)CF_3$ | H | 3-Cl | H | $CH_3$ | $CH_2CH_3$ | 1 | 2 | | |
| 83 | 2-$CH_3$ 4-$CF(CF_3)CF_3$ | H | 3-Br | H | $CH_3$ | $CH_2CH_3$ | 1 | 2 | | |
| 84 | 2-$CH_3$ 4-$CF(CF_3)CF_3$ | H | 3-$NO_2$ | H | $CH_3$ | $CH_2CH_3$ | 1 | 2 | | |
| 85 | 2-$CH_3$ 4-$CF(CF_3)CF_3$ | H | 3-$OSO_2CH_3$ | H | $CH_3$ | $CH_2CH_3$ | 1 | 2 | | |
| 86 | 2-$CH_3$ 4-$CF(CF_3)CF_3$ | H | 3-$SO_2CH_3$ | H | $CH_3$ | $CH_2CH_3$ | 1 | 2 | | |
| 87 | 2-$CH_3$ 4-$CF(CF_3)CF_3$ | H | 3-$SO_2CF_3$ | H | $CH_3$ | $CH_2CH_3$ | 1 | 2 | | |
| 88 | 2-$CH_3$ 4-$CF(CF_3)CF_3$ | H | 3-$CF_3$ | H | $CH_3$ | $CH_2CH_3$ | 1 | 2 | | |
| 89 | 2-$CH_3$ 4-(p-$CF_3$-phenyl) | H | 3-1 | H | $CH_3$ | $CH_2CH_3$ | 1 | 2 | | |
| 90 | 2-$CH_3$ 4-(p-$CF_3$-phenyl) | H | 3-Br | H | $CH_3$ | $CH_2CH_3$ | 1 | 2 | | |
| 91 | 2-$CH_3$ 4-(p-$CF_3$-phenyl) | H | 3-Cl | H | $CH_3$ | $CH_2CH_3$ | 1 | 2 | | |
| 92 | 2-$CH_3$ 4-(p-$CF_3$-phenyl) | H | 3-$NO_2$ | H | $CH_3$ | $CH_2CH_3$ | 1 | 2 | | |
| 93 | 2-$CH_3$ 4-(p-$CF_3$-phenyl) | H | 3-$OSO_2CH_3$ | H | $CH_3$ | $CH_2CH_3$ | 1 | 2 | | |
| 94 | 2-$CH_3$ 4-(p-$CF_3$-phenyl) | H | 3-$SO_2CH_3$ | H | $CH_3$ | $CH_2CH_3$ | 1 | 2 | | |
| 95 | 2-$CH_3$ 4-(p-$CF_3$-phenyl) | H | 3-$SO_2CF_3$ | H | $CH_3$ | $CH_2CH_3$ | 1 | 2 | | |
| 96 | 2-$CH_3$ 4-(p-$CF_3$-phenyl) | H | 3-$CF_3$ | H | $CH_3$ | $CH_2CH_3$ | 1 | 2 | | |
| 97 | 2-$CH_3$ 4-(3-$CF_3$-pyrazole-1-yl) | H | 3-1 | H | $CH_3$ | $CH_2CH_3$ | 1 | 2 | | |
| 98 | 2-$CH_3$ 4-(3-$CF_3$-pyrazole-1-yl) | H | 3-Cl | H | $CH_3$ | $CH_2CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 99 | 2-$CH_3$ 4-(3-$CF_3$-pyrazole-1-yl) | H | 3-Br | H | $CH_3$ | $CH_2CH_3$ | 1 | 2 | | |
| 100 | 2-$CH_3$ 4-(3-$CF_3$-pyrazole-1-yl) | H | 3-$NO_2$ | H | $CH_3$ | $CH_2CH_3$ | 1 | 2 | | |
| 101 | 2-$CH_3$ 4-(3-$CF_3$-pyrazole-1-yl) | H | 3-$OSO_2CH_3$ | H | $CH_3$ | $CH_2CH_3$ | 1 | 2 | | |
| 102 | 2-$CH_3$ 4-(3-$CF_3$-pyrazole-1-yl) | H | 3-$SO_2CH_3$ | H | $CH_3$ | $CH_2CH_3$ | 1 | 2 | | |
| 103 | 2-$CH_3$ 4-(3-$CF_3$-pyrazole-1-yl) | H | 3-$SO_2CF_3$ | H | $CH_3$ | $CH_2CH_3$ | 1 | 2 | | |
| 104 | 2-$CH_3$ 4-(3-$CF_3$-pyrazole-1-yl) | H | 3-$CF_3$ | H | $CH_3$ | $CH_2CH_3$ | 1 | 2 | | |
| 105 | 2-$CH_3$ 3-F 4-$CF(CF_3)CF_3$ | H | 3-1 | H | $CH_3$ | $CH_2CH_3$ | 1 | 0 | 4.71[a] | |
| 106 | 2-$CH_3$ 3-F 4-$CF(CF_3)CF_3$ | H | 3-Cl | H | $CH_3$ | $CH_2CH_3$ | 1 | 0 | | |
| 107 | 2-$CH_3$ 3-F 4-$CF(CF_3)CF_3$ | H | 3-Br | H | $CH_3$ | $CH_2CH_3$ | 1 | 0 | | |
| 108 | 2-$CH_3$ 3-F 4-$CF(CF_3)CF_3$ | H | 3-$NO_2$ | H | $CH_3$ | $CH_2CH_3$ | 1 | 0 | | |
| 109 | 2-$CH_3$ 3-F 4-$CF(CF_3)CF_3$ | H | 3-$OSO_2CH_3$ | H | $CH_3$ | $CH_2CH_3$ | 1 | 0 | | |
| 110 | 2-$CH_3$ 3-F 4-$CF(CF_3)CF_3$ | H | 3-$SO_2CH_3$ | H | $CH_3$ | $CH_2CH_3$ | 1 | 0 | | |
| 111 | 2-$CH_3$ 3-F 4-$CF(CF_3)CF_3$ | H | 3-$SO_2CF_3$ | H | $CH_3$ | $CH_2CH_3$ | 1 | 0 | | |
| 112 | 2-$CH_3$ 3-F 4-$CF(CF_3)CF_3$ | H | 3-$CF_3$ | H | $CH_3$ | $CH_2CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 113 | 2-$CH_3$ 4-(p-$CF_3$-phenyl) | H | 3-1 | H | $CH_3$ | $CH_2CH_3$ | 1 | 0 | | |
| 114 | 2-$CH_3$ 4-(p-$CF_3$-phenyl) | H | 3-Cl | H | $CH_3$ | $CH_2CH_3$ | 1 | 0 | | |
| 115 | 2-$CH_3$ 4-(p-$CF_3$-phenyl) | H | 3-Br | H | $CH_3$ | $CH_2CH_3$ | 1 | 0 | | |
| 116 | 2-$CH_3$ 4-(p-$CF_3$-phenyl) | H | 3-$NO_2$ | H | $CH_3$ | $CH_2CH_3$ | 1 | 0 | | |
| 117 | 2-$CH_3$ 4-(p-$CF_3$-phenyl) | H | 3-$OSO_2CH_3$ | H | $CH_3$ | $CH_2CH_3$ | 1 | 0 | | |
| 118 | 2-$CH_3$ 4-(p-$CF_3$-phenyl) | H | 3-$SO_2CH_3$ | H | $CH_3$ | $CH_2CH_3$ | 1 | 0 | | |
| 119 | 2-$CH_3$ 4-(p-$CF_3$-phenyl) | H | 3-$SO_2CF_3$ | H | $CH_3$ | $CH_2CH_3$ | 1 | 0 | | |
| 120 | 2-$CH_3$ 4-(p-$CF_3$-phenyl) | H | 3-$CF_3$ | H | $CH_3$ | $CH_2CH_3$ | 1 | 0 | | |
| 121 | 2-$CH_3$ 4-(3-$CF_3$-pyrazole-1-yl) | H | 3-1 | H | $CH_3$ | $CH_2CH_3$ | 1 | 0 | | |
| 122 | 2-$CH_3$ 4-(3-$CF_3$-pyrazole-1-yl) | H | 3-Cl | H | $CH_3$ | $CH_2CH_3$ | 1 | 0 | | |
| 123 | 2-$CH_3$ 4-(3-$CF_3$-pyrazole-1-yl) | H | 3-Br | H | $CH_3$ | $CH_2CH_3$ | 1 | 0 | | |
| 124 | 2-$CH_3$ 4-(3-$CF_3$-pyrazole-1-yl) | H | 3-$NO_2$ | H | $CH_3$ | $CH_2CH_3$ | 1 | 0 | | |
| 125 | 2-$CH_3$ 4-(3-$CF_3$-pyrazole-1-yl) | H | 3-$OSO_2CH_3$ | H | $CH_3$ | $CH_2CH_3$ | 1 | 0 | | |
| 126 | 2-$CH_3$ 4-(3-$CF_3$-pyrazole-1-yl) | H | 3-$SO_2CH_3$ | H | $CH_3$ | $CH_2CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 127 | 2-$CH_3$<br>4-(3-$CF_3$-pyrazole-1-yl) | H | 3-$SO_2CF_3$ | H | $CH_3$ | $CH_2CH_3$ | 1 | 0 | | |
| 128 | 2-$CH_3$<br>4-(3-$CF_3$-pyrazole-1-yl) | H | 3-$CF_3$ | H | $CH_3$ | $CH_2CH_3$ | 1 | 0 | | |
| 129 | 2-$CH_3$<br>4-$CF(CF_3)CF_3$ | H | 3-Br | H | $CH_3$ | $CH_3$ | 1 | 2 | | |
| 130 | 2-$CH_3$<br>3-F<br>4-$CF(CF_3)CF_3$ | H | 3-1 | H | $CH_3$ | $CH_2CH_3$ | 1 | 2 | 3.47[a] | |
| 131 | 2-$CH_3$<br>3-F<br>4-$CF(CF_3)CF_3$ | H | 3-Cl | H | $CH_3$ | $CH_2CH_3$ | 1 | 2 | | |
| 132 | 2-$CH_3$<br>3-F<br>4-$CF(CF_3)CF_3$ | H | 3-Br | H | $CH_3$ | $CH_2CH_3$ | 1 | 2 | | |
| 133 | 2-$CH_3$<br>3-F<br>4-$CF(CF_3)CF_3$ | H | 3-$NO_2$ | H | $CH_3$ | $CH_2CH_3$ | 1 | 2 | | |
| 134 | 2-$CH_3$<br>3-F<br>4-$CF(CF_3)CF_3$ | H | 3-$OSO_2CH_3$ | H | $CH_3$ | $CH_2CH_3$ | 1 | 2 | | |
| 135 | 2-$CH_3$<br>3-F<br>4-$CF(CF_3)CF_3$ | H | 3-$SO_2CH_3$ | H | $CH_3$ | $CH_2CH_3$ | 1 | 2 | | |
| 136 | 2-$CH_3$<br>3-F<br>4-$CF(CF_3)CF_3$ | H | 3-$SO_2CF_3$ | H | $CH_3$ | $CH_2CH_3$ | 1 | 2 | | |
| 137 | 2-$CH_3$<br>3-F<br>4-$CF(CF_3)CF_3$ | H | 3-$CF_3$ | H | $CH_3$ | $CH_2CH_3$ | 1 | 2 | | |
| 138 | 2-$CH_3$<br>3-Cl<br>4-$CF_2CF_3$ | H | 3-Br | H | $CH_3$ | $CH_2CH_3$ | 1 | 2 | 3.19[a] | |
| 139 | 2-$CH_3$<br>3-Cl<br>4-$CF_2CF_3$ | H | 3-Cl | H | $CH_3$ | $CH_2CH_3$ | 1 | 2 | | |
| 140 | 2-$CH_3$<br>3-Cl<br>4-$CF_2CF_3$ | H | 3-$NO_2$ | H | $CH_3$ | $CH_2CH_3$ | 1 | 2 | | |
| 141 | 2-$CH_3$<br>3-Cl<br>4-$CF_2CF_3$ | H | 3-$OSO_2CH_3$ | H | $CH_3$ | $CH_2CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 142 | 2-$CH_3$<br>3-Cl<br>4-$CF_2CF_3$ | H | 3-$SO_2CH_3$ | H | $CH_3$ | $CH_2CH_3$ | 1 | 2 | | |
| 143 | 2-$CH_3$<br>3-Cl<br>4-$CF_2CF_3$ | H | 3-$SO_2CF_3$ | H | $CH_3$ | $CH_2CH_3$ | 1 | 2 | | |
| 144 | 2-$CH_3$<br>3-Cl<br>4-$CF_2CF_3$ | H | 3-$CF_3$ | H | $CH_3$ | $CH_2CH_3$ | 1 | 2 | | |
| 145 | 2-$CH_3$<br>3-Cl<br>4-$CF_2CF_3$ | H | 3-1 | H | $CH_3$ | $CH_2CH_3$ | 1 | 2 | 3.28[a] | |
| 146 | 2-$CH_3$<br>3-F<br>4-$CF_2CF_3$ | H | 3-1 | H | $CH_3$ | $CH_3$ | 1 | 0 | 4.04[a] | |
| 147 | 2-$CH_3$<br>3-F<br>4-$CF_2CF_3$ | H | 3-Cl | H | $CH_3$ | $CH_3$ | 1 | 0 | | |
| 148 | 2-$CH_3$<br>3-F<br>4-$CF_2CF_3$ | H | 3-Br | H | $CH_3$ | $CH_3$ | 1 | 0 | | |
| 149 | 2-$CH_3$<br>3-F<br>4-$CF_2CF_3$ | H | 3-$NO_2$ | H | $CH_3$ | $CH_3$ | 1 | 0 | | |
| 150 | 2-$CH_3$<br>3-F<br>4-$CF_2CF_3$ | H | 3-<br>$OSO_2CH_3$ | H | $CH_3$ | $CH_3$ | 1 | 0 | | |
| 151 | 2-$CH_3$<br>3-F<br>4-$CF_2CF_3$ | H | 3-$SO_2CH_3$ | H | $CH_3$ | $CH_3$ | 1 | 0 | | |
| 152 | 2-$CH_3$<br>3-F<br>4-$CF_2CF_3$ | H | 3-$SO_2CF_3$ | H | $CH_3$ | $CH_3$ | 1 | 0 | | |
| 153 | 2-$CH_3$<br>3-F<br>4-$CF_2CF_3$ | H | 3-$CF_3$ | H | $CH_3$ | $CH_3$ | 1 | 0 | | |
| 154 | 2-$CH_3$<br>4-$CF(CF_3)$<br>$CF_2Br$ | H | 3-1 | H | $CH_3$ | $CH_2CH_3$ | 1 | 0 | 4.68[a]<br>4.71[c] | 114 |
| 155 | 2-$CH_3$<br>4-$CF(CF_3)$<br>$CF_2Br$ | H | 3-Cl | H | $CH_3$ | $CH_2CH_3$ | 1 | 0 | | |
| 156 | 2-$CH_3$<br>4-$CF(CF_3)$<br>$CF_2Br$ | H | 3-Br | H | $CH_3$ | $CH_2CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 157 | 2-$CH_3$ 4-$CF(CF_3)CF_2Br$ | H | 3-$NO_2$ | H | $CH_3$ | $CH_2CH_3$ | 1 | 0 | | |
| 158 | 2-$CH_3$ 4-$CF(CF_3)CF_2Br$ | H | 3-$OSO_2CH_3$ | H | $CH_3$ | $CH_2CH_3$ | 1 | 0 | | |
| 159 | 2-$CH_3$ 4-$CF(CF_3)CF_2Br$ | H | 3-$SO_2CH_3$ | H | $CH_3$ | $CH_2CH_3$ | 1 | 0 | | |
| 160 | 2-$CH_3$ 4-$CF(CF_3)CF_2Br$ | H | 3-$SO_2CF_3$ | H | $CH_3$ | $CH_2CH_3$ | 1 | 0 | | |
| 161 | 2-$CH_3$ 4-$CF(CF_3)CF_2Br$ | H | 3-$CF_3$ | H | $CH_3$ | $CH_2CH_3$ | 1 | 0 | | |
| 162 | 2-$CH_3$ 3-F 4-$CF_2CF_3$ | H | 3-1 | H | $CH_3$ | $CH_3$ | 1 | 2 | 2.97[a] | |
| 163 | 2-$CH_3$ 3-F 4-$CF_2CF_3$ | H | 3-Cl | H | $CH_3$ | $CH_3$ | 1 | 2 | | |
| 164 | 2-$CH_3$ 3-F 4-$CF_2CF_3$ | H | 3-Br | H | $CH_3$ | $CH_3$ | 1 | 2 | | |
| 165 | 2-$CH_3$ 3-F 4-$CF_2CF_3$ | H | 3-$NO_2$ | H | $CH_3$ | $CH_3$ | 1 | 2 | | |
| 166 | 2-$CH_3$ 3-F 4-$CF_2CF_3$ | H | 3-$OSO_2CH_3$ | H | $CH_3$ | $CH_3$ | 1 | 2 | | |
| 167 | 2-$CH_3$ 3-F 4-$CF_2CF_3$ | H | 3-$SO_2CH_3$ | H | $CH_3$ | $CH_3$ | 1 | 2 | | |
| 168 | 2-$CH_3$ 3-F 4-$CF_2CF_3$ | H | 3-$SO_2CF_3$ | H | $CH_3$ | $CH_3$ | 1 | 2 | | |
| 169 | 2-$CH_3$ 3-F 4-$CF_2CF_3$ | H | 3-$CF_3$ | H | $CH_3$ | $CH_3$ | 1 | 2 | | |
| 170 | 2-$CH_3$ 3-Cl 4-$CF_2CF_3$ | H | 3-1 | H | $CH_3$ | $CH_3$ | 1 | 0 | 4.2[a] | |
| 171 | 2-$CH_3$ 3-Cl 4-$CF_2CF_3$ | H | 3-Cl | H | $CH_3$ | $CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 172 | 2-$CH_3$ 3-Cl 4-$CF_2CF_3$ | H | 3-Br | H | $CH_3$ | $CH_3$ | 1 | 0 | | |
| 173 | 2-$CH_3$ 3-Cl 4-$CF_2CF_3$ | H | 3-$NO_2$ | H | $CH_3$ | $CH_3$ | 1 | 0 | | |
| 174 | 2-$CH_3$ 3-Cl 4-$CF_2CF_3$ | H | 3-$OSO_2CH_3$ | H | $CH_3$ | $CH_3$ | 1 | 0 | | |
| 175 | 2-$CH_3$ 3-Cl 4-$CF_2CF_3$ | H | 3-$SO_2CH_3$ | H | $CH_3$ | $CH_3$ | 1 | 0 | | |
| 176 | 2-$CH_3$ 3-Cl 4-$CF_2CF_3$ | H | 3-$SO_2CF_3$ | H | $CH_3$ | $CH_3$ | 1 | 0 | | |
| 177 | 2-$CH_3$ 3-Cl 4-$CF_2CF_3$ | H | 3-$CF_3$ | H | $CH_3$ | $CH_3$ | 1 | 0 | | |
| 178 | 2-$CH_3$ 3-F 4-$CF(CF_3)CF_3$ | H | 3-1 | H | $CH_3$ | $CH_3$ | 1 | 2 | 3.27[a] | |
| 179 | 2-$CH_3$ 3-F 4-$CF(CF_3)CF_3$ | H | 3-Cl | H | $CH_3$ | $CH_3$ | 1 | 2 | | |
| 180 | 2-$CH_3$ 3-F 4-$CF(CF_3)CF_3$ | H | 3-Br | H | $CH_3$ | $CH_3$ | 1 | 2 | | |
| 181 | 2-$CH_3$ 3-F 4-$CF(CF_3)CF_3$ | H | 3-$NO_2$ | H | $CH_3$ | $CH_3$ | 1 | 2 | | |
| 182 | 2-$CH_3$ 3-F 4-$CF(CF_3)CF_3$ | H | 3-$OSO_2CH_3$ | H | $CH_3$ | $CH_3$ | 1 | 2 | | |
| 183 | 2-$CH_3$ 3-F 4-$CF(CF_3)CF_3$ | H | 3-$SO_2CH_3$ | H | $CH_3$ | $CH_3$ | 1 | 2 | | |
| 184 | 2-$CH_3$ 3-F 4-$CF(CF_3)CF_3$ | H | 3-$SO_2CF_3$ | H | $CH_3$ | $CH_3$ | 1 | 2 | | |
| 185 | 2-$CH_3$ 3-F 4-$CF(CF_3)CF_3$ | H | 3-$CF_3$ | H | $CH_3$ | $CH_3$ | 1 | 2 | | |
| 186 | 2-$CH_3$ 3-Cl 4-$CF_2CF_3$ | H | 3-1 | H | $CH_3$ | $CH_3$ | 1 | 2 | 3.1[a] | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 187 | 2-$CH_3$ 3-Cl 4-$CF_2CF_3$ | H | 3-Cl | H | $CH_3$ | $CH_3$ | 1 | 2 | | |
| 188 | 2-$CH_3$ 3-Cl 4-$CF_2CF_3$ | H | 3-Br | H | $CH_3$ | $CH_3$ | 1 | 2 | | |
| 189 | 2-$CH_3$ 3-Cl 4-$CF_2CF_3$ | H | 3-$NO_2$ | H | $CH_3$ | $CH_3$ | 1 | 2 | | |
| 190 | 2-$CH_3$ 3-Cl 4-$CF_2CF_3$ | H | 3-$OSO_2CH_3$ | H | $CH_3$ | $CH_3$ | 1 | 2 | | |
| 191 | 2-$CH_3$ 3-Cl 4-$CF_2CF_3$ | H | 3-$SO_2CH_3$ | H | $CH_3$ | $CH_3$ | 1 | 2 | | |
| 192 | 2-$CH_3$ 3-Cl 4-$CF_2CF_3$ | H | 3-$SO_2CF_3$ | H | $CH_3$ | $CH_3$ | 1 | 2 | | |
| 193 | 2-$CH_3$ 3-Cl 4-$CF_2CF_3$ | H | 3-$CF_3$ | H | $CH_3$ | $CH_3$ | 1 | 2 | | |
| 194 | 2-$CH_3$ 4-$CF(CF_3)CF_2Br$ | H | 3-1 | H | $CH_3$ | $CH_2CH_3$ | 1 | 2 | 3.49[a] 3.47[c] | 133 |
| 195 | 2-$CH_3$ 4-$CF(CF_3)CF_2Br$ | H | 3-Cl | H | $CH_3$ | $CH_2CH_3$ | 1 | 2 | | |
| 196 | 2-$CH_3$ 4-$CF(CF_3)CF_2Br$ | H | 3-Br | H | $CH_3$ | $CH_2CH_3$ | 1 | 2 | | |
| 197 | 2-$CH_3$ 4-$CF(CF_3)CF_2Br$ | H | 3-$NO_2$ | H | $CH_3$ | $CH_2CH_3$ | 1 | 2 | | |
| 198 | 2-$CH_3$ 4-$CF(CF_3)CF_2Br$ | H | 3-$OSO_2CH_3$ | H | $CH_3$ | $CH_2CH_3$ | 1 | 2 | | |
| 199 | 2-$CH_3$ 4-$CF(CF_3)CF_2Br$ | H | 3-$SO_2CH_3$ | H | $CH_3$ | $CH_2CH_3$ | 1 | 2 | | |
| 200 | 2-$CH_3$ 4-$CF(CF_3)CF_2Br$ | H | 3-$SO_2CF_3$ | H | $CH_3$ | $CH_2CH_3$ | 1 | 2 | | |
| 201 | 2-$CH_3$ 4-$CF(CF_3)CF_2Br$ | H | 3-$CF_3$ | H | $CH_3$ | $CH_2CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 202 | 2-$CH_3$ 4-CF($CHF_2$) $CF_3$ | H | 3-1 | H | $CH_3$ | $CH_2CH_3$ | 1 | 0 | 4.2[a] 4.2[c] | 187 |
| 203 | 2-$CH_3$ 4-CF($CHF_2$) $CF_3$ | H | 3-Cl | H | $CH_3$ | $CH_2CH_3$ | 1 | 0 | | |
| 204 | 2-$CH_3$ 4-CF($CHF_2$) $CF_3$ | H | 3-Br | H | $CH_3$ | $CH_2CH_3$ | 1 | 0 | | |
| 205 | 2-$CH_3$ 4-CF($CHF_2$) $CF_3$ | H | 3-$NO_2$ | H | $CH_3$ | $CH_2CH_3$ | 1 | 0 | | |
| 206 | 2-$CH_3$ 4-CF($CHF_2$) $CF_3$ | H | 3-$OSO_2CH_3$ | H | $CH_3$ | $CH_2CH_3$ | 1 | 0 | | |
| 207 | 2-$CH_3$ 4-CF($CHF_2$) $CF_3$ | H | 3-$SO_2CH_3$ | H | $CH_3$ | $CH_2CH_3$ | 1 | 0 | | |
| 208 | 2-$CH_3$ 4-CF($CHF_2$) $CF_3$ | H | 3-$SO_2CF_3$ | H | $CH_3$ | $CH_2CH_3$ | 1 | 0 | | |
| 209 | 2-$CH_3$ 4-CF($CHF_2$) $CF_3$ | H | 3-$CF_3$ | H | $CH_3$ | $CH_2CH_3$ | 1 | 0 | | |
| 210 | 2-$CH_3$ 4-CH($CHF_2$) $CF_3$ | H | 3-1 | H | $CH_3$ | $CH_2CH_3$ | 1 | 0 | 3.63[a] 3.62[c] | 105 |
| 211 | 2-$CH_3$ 4-CH($CHF_2$) $CF_3$ | H | 3-Cl | H | $CH_3$ | $CH_2CH_3$ | 1 | 0 | | |
| 212 | 2-$CH_3$ 4-CH($CHF_2$) $CF_3$ | H | 3-Br | H | $CH_3$ | $CH_2CH_3$ | 1 | 0 | | |
| 213 | 2-$CH_3$ 4-CH($CHF_2$) $CF_3$ | H | 3-$NO_2$ | H | $CH_3$ | $CH_2CH_3$ | 1 | 0 | | |
| 214 | 2-$CH_3$ 4-CH($CHF_2$) $CF_3$ | H | 3-$OSO_2CH_3$ | H | $CH_3$ | $CH_2CH_3$ | 1 | 0 | | |
| 215 | 2-$CH_3$ 4-CH ($CHF_2$)$CF_3$ | H | 3-$SO_2CH_3$ | H | $CH_3$ | $CH_2CH_3$ | 1 | 0 | | |
| 216 | 2-$CH_3$ 4-CH($CHF_2$) $CF_3$ | H | 3-$SO_2CF_3$ | H | $CH_3$ | $CH_2CH_3$ | 1 | 0 | | |
| 217 | 2-$CH_3$ 4-CH($CHF_2$) $CF_3$ | H | 3-$CF_3$ | H | $CH_3$ | $CH_2CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 218 | 2-$CH_3$<br>4-CF($CHF_2$)<br>$CF_3$ | H | 3-1 | H | $CH_3$ | $CH_2CH_3$ | 1 | 0 | 3.96[a]<br>3.96[c] | 105 |
| 219 | 2-F<br>4-CH($CHF_2$)<br>$CF_3$ | H | 3-1 | H | $CH_3$ | $CH_2CH_3$ | 1 | 0 | 3.57[a]<br>3.57[c] | 144 |
| 220 | 2-F<br>4-CH($CHF_2$)<br>$CF_3$ | H | 3-Cl | H | $CH_3$ | $CH_2CH_3$ | 1 | 0 | | |
| 221 | 2-F<br>4-CH($CHF_2$)<br>$CF_3$ | H | 3-Br | H | $CH_3$ | $CH_2CH_3$ | 1 | 0 | | |
| 222 | 2-F<br>4-CH($CHF_2$)<br>$CF_3$ | H | 3-$NO_2$ | H | $CH_3$ | $CH_2CH_3$ | 1 | 0 | | |
| 223 | 2-F<br>4-CH($CHF_2$)<br>$CF_3$ | H | 3-<br>$OSO_2CH_3$ | H | $CH_3$ | $CH_2CH_3$ | 1 | 0 | | |
| 224 | 2-F<br>4-CH($CHF_2$)<br>$CF_3$ | H | 3-$SO_2CH_3$ | H | $CH_3$ | $CH_2CH_3$ | 1 | 0 | | |
| 225 | 2-F<br>4-CH($CHF_2$)<br>$CF_3$ | H | 3-$SO_2CF_3$ | H | $CH_3$ | $CH_2CH_3$ | 1 | 0 | | |
| 226 | 2-F<br>4-CH($CHF_2$)<br>$CF_3$ | H | 3-$CF_3$ | H | $CH_3$ | $CH_2CH_3$ | 1 | 0 | | |
| 227 | 2-Br<br>4-CH($CHF_2$)<br>$CF_3$ | H | 3-1 | H | $CH_3$ | $CH_3$ | 1 | 0 | 3.51[a]<br>3.54[c] | 167 |
| 228 | 2-Br<br>4-CH($CHF_2$)<br>$CF_3$ | H | 3-Cl | H | $CH_3$ | $CH_3$ | 1 | 0 | | |
| 229 | 2-Br<br>4-CH($CHF_2$)<br>$CF_3$ | H | 3-Br | H | $CH_3$ | $CH_3$ | 1 | 0 | | |
| 230 | 2-Br<br>4-CH($CHF_2$)<br>$CF_3$ | H | 3-$NO_2$ | H | $CH_3$ | $CH_3$ | 1 | 0 | | |
| 231 | 2-Br<br>4-CH($CHF_2$)<br>$CF_3$ | H | 3-<br>$OSO_2CH_3$ | H | $CH_3$ | $CH_3$ | 1 | 0 | | |
| 232 | 2-Br<br>4-CH($CHF_2$)<br>$CF_3$ | H | 3-$SO_2CH_3$ | H | $CH_3$ | $CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 233 | 2-Br 4-CH($CHF_2$) $CF_3$ | H | 3-$SO_2CF_3$ | H | $CH_3$ | $CH_3$ | 1 | 0 | | |
| 234 | 2-Br 4-CH($CHF_2$) $CF_3$ | H | 3-$CF_3$ | H | $CH_3$ | $CH_3$ | 1 | 0 | | |
| 235 | 2-Cl 4-CH($CHF_2$) $CF_3$ | H | 3-1 | H | $CH_3$ | $CH_2CH_3$ | 1 | 0 | 3.76[a] 3.79[c] | 165 |
| 236 | 2-Cl 4-CH ($CHF_2$)$CF_3$ | H | 3-Cl | H | $CH_3$ | $CH_2CH_3$ | 1 | 0 | | |
| 237 | 2-Cl 4-CH($CHF_2$) $CF_3$ | H | 3-Br | H | $CH_3$ | $CH_2CH_3$ | 1 | 0 | | |
| 238 | 2-Cl 4-CH($CHF_2$) $CF_3$ | H | 3-$NO_2$ | H | $CH_3$ | $CH_2CH_3$ | 1 | 0 | | |
| 239 | 2-Cl 4-CH($CHF_2$) $CF_3$ | H | 3-$OSO_2CH_3$ | H | $CH_3$ | $CH_2CH_3$ | 1 | 0 | | |
| 240 | 2-Cl 4-CH($CHF_2$) $CF_3$ | H | 3-$SO_2CH_3$ | H | $CH_3$ | $CH_2CH_3$ | 1 | 0 | | |
| 241 | 2-Cl 4-CH($CHF_2$) $CF_3$ | H | 3-$SO_2CF_3$ | H | $CH_3$ | $CH_2CH_3$ | 1 | 0 | | |
| 242 | 2-Cl 4-CH($CHF_2$) $CF_3$ | H | 3-$CF_3$ | H | $CH_3$ | $CH_2CH_3$ | 1 | 0 | | |
| 243 | 2-Br 4-CH($CHF_2$) $CF_3$ | H | 3-1 | H | $CH_3$ | $CH_2CH_3$ | 1 | 0 | 3.78[a] 3.8[c] | 160 |
| 244 | 2-Br 4-CH($CHF_2$) $CF_3$ | H | 3-Cl | H | $CH_3$ | $CH_2CH_3$ | 1 | 0 | | |
| 245 | 2-Br 4-CH($CHF_2$) $CF_3$ | H | 3-Br | H | $CH_3$ | $CH_2CH_3$ | 1 | 0 | | |
| 246 | 2-Br 4-CH($CHF_2$) $CF_3$ | H | 3-$NO_2$ | H | $CH_3$ | $CH_2CH_3$ | 1 | 0 | | |
| 247 | 2-Br 4-CH($CHF_2$) $CF_3$ | H | 3-$OSO_2CH_3$ | H | $CH_3$ | $CH_2CH_3$ | 1 | 0 | | |
| 248 | 2-Br 4-CH($CHF_2$) $CF_3$ | H | 3-$SO_2CH_3$ | H | $CH_3$ | $CH_2CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 249 | 2-Br 4-CH($CHF_2$) $CF_3$ | H | 3-$SO_2CF_3$ | H | $CH_3$ | $CH_2CH_3$ | 1 | 0 | | |
| 250 | 2-Br 4-CH($CHF_2$) $CF_3$ | H | 3-$CF_3$ | H | $CH_3$ | $CH_2CH_3$ | 1 | 0 | | |
| 251 | 2-Cl 4-CF($CHF_2$) $CF_3$ | H | 3-1 | H | $CH_3$ | $CH_2CH_3$ | 1 | 2 | 3.12[a] 3.18[c] | 132-135 |
| 252 | 2-Cl 4-CF($CHF_2$) $CF_3$ | H | 3-Cl | H | $CH_3$ | $CH_2CH_3$ | 1 | 2 | | |
| 253 | 2-Cl 4-CF($CHF_2$) $CF_3$ | H | 3-Br | H | $CH_3$ | $CH_2CH_3$ | 1 | 2 | | |
| 254 | 2-Cl 4-CF($CHF_2$) $CF_3$ | H | 3-$NO_2$ | H | $CH_3$ | $CH_2CH_3$ | 1 | 2 | | |
| 255 | 2-Cl 4-CF($CHF_2$) $CF_3$ | H | 3-$OSO_2CH_3$ | H | $CH_3$ | $CH_2CH_3$ | 1 | 2 | | |
| 256 | 2-Cl 4-CF ($CHF_2$)$CF_3$ | H | 3-$SO_2CH_3$ | H | $CH_3$ | $CH_2CH_3$ | 1 | 2 | | |
| 257 | 2-Cl 4-CF($CHF_2$) $CF_3$ | H | 3-$SO_2CF_3$ | H | $CH_3$ | $CH_2CH_3$ | 1 | 2 | | |
| 258 | 2-Cl 4-CF($CHF_2$) $CF_3$ | H | 3-$CF_3$ | H | $CH_3$ | $CH_2CH_3$ | 1 | 2 | | |
| 259 | 2-Cl 4-CH($CHF_2$) $CF_3$ | H | 3-1 | H | $CH_3$ | $CH_3$ | 1 | 2 | 2.69[a] 2.72[c] | 116-120 |
| 260 | 2-Cl 4-CH($CHF_2$) $CF_3$ | H | 3-Cl | H | $CH_3$ | $CH_3$ | 1 | 2 | | |
| 261 | 2-Cl 4-CH ($CHF_2$)$CF_3$ | H | 3-Br | H | $CH_3$ | $CH_3$ | 1 | 2 | | |
| 262 | 2-Cl 4-CH($CHF_2$) $CF_3$ | H | 3-$NO_2$ | H | $CH_3$ | $CH_3$ | 1 | 2 | | |
| 263 | 2-Cl 4-CH($CHF_2$) $CF_3$ | H | 3-$OSO_2CH_3$ | H | $CH_3$ | $CH_3$ | 1 | 2 | | |
| 264 | 2-Cl 4-CH($CHF_2$) $CF_3$ | H | 3-$SO_2CH_3$ | H | $CH_3$ | $CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 265 | 2-Cl 4-CH($CHF_2$) $CF_3$ | H | 3-$SO_2CF_3$ | H | $CH_3$ | $CH_3$ | 1 | 2 | | |
| 266 | 2-Cl 4-CH($CHF_2$) $CF_3$ | H | 3-$CF_3$ | H | $CH_3$ | $CH_3$ | 1 | 2 | | |
| 267 | 2-Br 4-CF($CHF_2$) $CF_3$ | H | 3-Br | H | $CH_3$ | $CH_3$ | 1 | 0 | 3.81[a] | 138-142 |
| 268 | 2-Br 4-CF($CHF_2$) $CF_3$ | H | 3-Cl | H | $CH_3$ | $CH_3$ | 1 | 0 | | |
| 269 | 2-Br 4-CF($CHF_2$) $CF_3$ | H | 3-1 | H | $CH_3$ | $CH_3$ | 1 | 0 | | |
| 270 | 2-Br 4-CF($CHF_2$) $CF_3$ | H | 3-$NO_2$ | H | $CH_3$ | $CH_3$ | 1 | 0 | | |
| 271 | 2-Br 4-CF($CHF_2$) $CF_3$ | H | 3-$OSO_2CH_3$ | H | $CH_3$ | $CH_3$ | 1 | 0 | | |
| 272 | 2-Br 4-CF($CHF_2$) $CF_3$ | H | 3-$SO_2CH_3$ | H | $CH_3$ | $CH_3$ | 1 | 0 | | |
| 273 | 2-Br 4-CF($CHF_2$) $CF_3$ | H | 3-$SO_2CF_3$ | H | $CH_3$ | $CH_3$ | 1 | 0 | | |
| 274 | 2-Br 4-CF($CHF_2$) $CF_3$ | H | 3-$CF_3$ | H | $CH_3$ | $CH_3$ | 1 | 0 | | |
| 275 | 2-Br 4-CH($CHF_2$) $CF_3$ | H | 3-1 | H | $CH_3$ | $CH_3$ | 1 | 2 | 2.71[a] 2.76[c] | 198 |
| 276 | 2-Br 4-CH($CHF_2$) $CF_3$ | H | 3-Cl | H | $CH_3$ | $CH_3$ | 1 | 2 | | |
| 277 | 2-Br 4-CH($CHF_2$) $CF_3$ | H | 3-Br | H | $CH_3$ | $CH_3$ | 1 | 2 | | |
| 278 | 2-Br 4-CH($CHF_2$) $CF_3$ | H | 3-$NO_2$ | H | $CH_3$ | $CH_3$ | 1 | 2 | | |
| 279 | 2-Br 4-CH($CHF_2$) $CF_3$ | H | 3-$OSO_2CH_3$ | H | $CH_3$ | $CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 280 | 2-Br 4-CH($CHF_2$) $CF_3$ | H | 3-$SO_2CH_3$ | H | $CH_3$ | $CH_3$ | 1 | 2 | | |
| 281 | 2-Br 4-CH($CHF_2$) $CF_3$ | H | 3-$SO_2CF_3$ | H | $CH_3$ | $CH_3$ | 1 | 2 | | |
| 282 | 2-Br 4-CH($CHF_2$) $CF_3$ | H | 3-$CF_3$ | H | $CH_3$ | $CH_3$ | 1 | 2 | | |
| 283 | 2-F 4-CF($CHF_2$) $CF_3$ | H | 3-1 | H | $CH_3$ | $CH_3$ | 1 | 2 | 2.72[a] 2.76[c] | 124-126 |
| 284 | 2-F 4-CF($CHF_2$) $CF_3$ | H | 3-Cl | H | $CH_3$ | $CH_3$ | 1 | 2 | | |
| 285 | 2-F 4-CF($CHF_2$) $CF_3$ | H | 3-Br | H | $CH_3$ | $CH_3$ | 1 | 2 | | |
| 286 | 2-F 4-CF($CHF_2$) $CF_3$ | H | 3-$NO_2$ | H | $CH_3$ | $CH_3$ | 1 | 2 | | |
| 287 | 2-F 4-CF($CHF_2$) $CF_3$ | H | 3- $OSO_2CH_3$ | H | $CH_3$ | $CH_3$ | 1 | 2 | | |
| 288 | 2-F 4-CF($CHF_2$) $CF_3$ | H | 3-$SO_2CH_3$ | H | $CH_3$ | $CH_3$ | 1 | 2 | | |
| 289 | 2-F 4-CF($CHF_2$) $CF_3$ | H | 3-$SO_2CF_3$ | H | $CH_3$ | $CH_3$ | 1 | 2 | | |
| 290 | 2-F 4-CF($CHF_2$) $CF_3$ | H | 3-$CF_3$ | H | $CH_3$ | $CH_3$ | 1 | 2 | | |
| 291 | 2-F 4-CH($CHF_2$) $CF_3$ | H | 3-1 | H | $CH_3$ | $CH_3$ | 1 | 2 | 2.5[a] 2.54[c] | 144-147 |
| 292 | 2-F 4-CH($CHF_2$) $CF_3$ | H | 3-Cl | H | $CH_3$ | $CH_3$ | 1 | 2 | | |
| 293 | 2-F 4-CH($CHF_2$) $CF_3$ | H | 3-Br | H | $CH_3$ | $CH_3$ | 1 | 2 | | |
| 294 | 2-F 4-CH($CHF_2$) $CF_3$ | H | 3-$NO_2$ | H | $CH_3$ | $CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 295 | 2-F 4-$CH(CHF_2)CF_3$ | H | 3-$OSO_2CH_3$ | H | $CH_3$ | $CH_3$ | 1 | 2 | | |
| 296 | 2-F 4-$CH(CHF_2)CF_3$ | H | 3-$SO_2CH_3$ | H | $CH_3$ | $CH_3$ | 1 | 2 | | |
| 297 | 2-F 4-$CH(CHF_2)CF_3$ | H | 3-$SO_2CF_3$ | H | $CH_3$ | $CH_3$ | 1 | 2 | | |
| 298 | 2-F 4-$CH(CHF_2)CF_3$ | H | 3-$CF_3$ | H | $CH_3$ | $CH_3$ | 1 | 2 | | |
| 299 | 2-Br 4-$CH(CHF_2)CF_3$ | H | 3-1 | H | $CH_3$ | $CH_2CH_3$ | 1 | 2 | 2.87[a] 2.92[c] | 122 |
| 300 | 2-Br 4-$CH(CHF_2)CF_3$ | H | 3-Cl | H | $CH_3$ | $CH_2CH_3$ | 1 | 2 | | |
| 301 | 2-Br 4-$CH(CHF_2)CF_3$ | H | 3-Br | H | $CH_3$ | $CH_2CH_3$ | 1 | 2 | | |
| 302 | 2-Br 4-$CH(CHF_2)CF_3$ | H | 3-$NO_2$ | H | $CH_3$ | $CH_2CH_3$ | 1 | 2 | | |
| 303 | 2-Br 4-$CH(CHF_2)CF_3$ | H | 3-$OSO_2CH_3$ | H | $CH_3$ | $CH_2CH_3$ | 1 | 2 | | |
| 304 | 2-Br 4-$CH(CHF_2)CF_3$ | H | 3-$SO_2CH_3$ | H | $CH_3$ | $CH_2CH_3$ | 1 | 2 | | |
| 305 | 2-Br 4-$CH(CHF_2)CF_3$ | H | 3-$SO_2CF_3$ | H | $CH_3$ | $CH_2CH_3$ | 1 | 2 | | |
| 306 | 2-Br 4-$CH(CHF_2)CF_3$ | H | 3-$CF_3$ | H | $CH_3$ | $CH_2CH_3$ | 1 | 2 | | |
| 307 | 2-F 4-$CF(CHF_2)CF_3$ | H | 3-1 | H | $CH_3$ | $CH_2CH_3$ | 1 | 2 | 2.89[a] 2.94[c] | 142-144 |
| 308 | 2-F 4-$CF(CHF_2)CF_3$ | H | 3-Cl | H | $CH_3$ | $CH_2CH_3$ | 1 | 2 | | |
| 309 | 2-F 4-$CF(CHF_2)CF_3$ | H | 3-Br | H | $CH_3$ | $CH_2CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 310 | 2-F<br>4-CF($CHF_2$)<br>$CF_3$ | H | 3-$NO_2$ | H | $CH_3$ | $CH_2CH_3$ | 1 | 2 | | |
| 311 | 2-F<br>4-CF($CHF_2$)<br>$CF_3$ | H | 3-<br>$OSO_2CH_3$ | H | $CH_3$ | $CH_2CH_3$ | 1 | 2 | | |
| 312 | 2-F<br>4-CF($CHF_2$)<br>$CF_3$ | H | 3-$SO_2CH_3$ | H | $CH_3$ | $CH_2CH_3$ | 1 | 2 | | |
| 313 | 2-F<br>4-CF($CHF_2$)<br>$CF_3$ | H | 3-$SO_2CF_3$ | H | $CH_3$ | $CH_2CH_3$ | 1 | 2 | | |
| 314 | 2-F<br>4-CF($CHF_2$)<br>$CF_3$ | H | 3-$CF_3$ | H | $CH_3$ | $CH_2CH_3$ | 1 | 2 | | |
| 315 | 2-F<br>4-CH($CHF_2$)<br>$CF_3$ | H | 3-1 | H | $CH_3$ | $CH_2CH_3$ | 1 | 2 | 2.65[a]<br>2.69[c] | 128 |
| 316 | 2-F<br>4-CH($CHF_2$)<br>$CF_3$ | H | 3-Cl | H | $CH_3$ | $CH_2CH_3$ | 1 | 2 | | |
| 317 | 2-F<br>4-CH($CHF_2$)<br>$CF_3$ | H | 3-Br | H | $CH_3$ | $CH_2CH_3$ | 1 | 2 | | |
| 318 | 2-F<br>4-CH($CHF_2$)<br>$CF_3$ | H | 3-$NO_2$ | H | $CH_3$ | $CH_2CH_3$ | 1 | 2 | | |
| 319 | 2-F<br>4-CH($CHF_2$)<br>$CF_3$ | H | 3-<br>$OSO_2CH_3$ | H | $CH_3$ | $CH_2CH_3$ | 1 | 2 | | |
| 320 | 2-F<br>4-CH($CHF_2$)<br>$CF_3$ | H | 3-$SO_2CH_3$ | H | $CH_3$ | $CH_2CH_3$ | 1 | 2 | | |
| 321 | 2-F<br>4-CH($CHF_2$)<br>$CF_3$ | H | 3-$SO_2CF_3$ | H | $CH_3$ | $CH_2CH_3$ | 1 | 2 | | |
| 322 | 2-F<br>4-CH($CHF_2$)<br>$CF_3$ | H | 3-$CF_3$ | H | $CH_3$ | $CH_2CH_3$ | 1 | 2 | | |
| 323 | 2-$CH_3$<br>3-F<br>4-$CF_2CF_3$ | H | 3-1 | H | $CH_3$ | $CH_3$ | 1 | 1 | 2.5[a] | 71-74 |
| 324 | 2-$CH_3$<br>3-F<br>4-$CF_2CF_3$ | H | 3-Cl | H | $CH_3$ | $CH_3$ | 1 | 1 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 325 | 2-$CH_3$<br>3-F<br>4-$CF_2CF_3$ | H | 3-Br | H | $CH_3$ | $CH_3$ | 1 | 1 | | |
| 326 | 2-$CH_3$<br>3-F<br>4-$CF_2CF_3$ | H | 3-$NO_2$ | H | $CH_3$ | $CH_3$ | 1 | 1 | | |
| 327 | 2-$CH_3$<br>3-F<br>4-$CF_2CF_3$ | H | 3-<br>$OSO_2CH_3$ | H | $CH_3$ | $CH_3$ | 1 | 1 | | |
| 328 | 2-$CH_3$<br>3-F<br>4-$CF_2CF_3$ | H | 3-$SO_2CH_3$ | H | $CH_3$ | $CH_3$ | 1 | 1 | | |
| 329 | 2-$CH_3$<br>3-F<br>4-$CF_2CF_3$ | H | 3-$SO_2CF_3$ | H | $CH_3$ | $CH_3$ | 1 | 1 | | |
| 330 | 2-$CH_3$<br>3-F<br>4-$CF_2CF_3$ | H | 3-$CF_3$ | H | $CH_3$ | $CH_3$ | 1 | 1 | | |
| 331 | 2-$CH_3$<br>3-F<br>4-$CF(CF_3)CF_3$ | H | 3-1 | H | $CH_3$ | $CH_3$ | 1 | 1 | 2.78[a] | |
| 332 | 2-$CH_3$<br>3-F<br>4-$CF(CF_3)CF_3$ | H | 3-Cl | H | $CH_3$ | $CH_3$ | 1 | 1 | | |
| 333 | 2-$CH_3$<br>3-F<br>4-$CF(CF_3)CF_3$ | H | 3-Br | H | $CH_3$ | $CH_3$ | 1 | 1 | | |
| 334 | 2-$CH_3$<br>3-F<br>4-$CF(CF_3)CF_3$ | H | 3-$NO_2$ | H | $CH_3$ | $CH_3$ | 1 | 1 | | |
| 335 | 2-$CH_3$<br>3-F<br>4-$CF(CF_3)CF_3$ | H | 3-<br>$OSO_2CH_3$ | H | $CH_3$ | $CH_3$ | 1 | 1 | | |
| 336 | 2-$CH_3$<br>3-F<br>4-$CF(CF_3)CF_3$ | H | 3-$SO_2CH_3$ | H | $CH_3$ | $CH_3$ | 1 | 1 | | |
| 337 | 2-$CH_3$<br>3-F<br>4-$CF(CF_3)CF_3$ | H | 3-$SO_2CF_3$ | H | $CH_3$ | $CH_3$ | 1 | 1 | | |
| 338 | 2-$CH_3$<br>3-F<br>4-$CF(CF_3)CF_3$ | H | 3-$CF_3$ | H | $CH_3$ | $CH_3$ | 1 | 1 | | |
| 339 | 2-$CH_3$<br>3-F<br>4-$CF(CF_3)CF_3$ | H | 3-1 | H | $CH_3$ | $CH_2CH_3$ | 1 | 1 | 2.97[a] | 210 |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 340 | 2-$CH_3$<br>3-F<br>4-$CF(CF_3)CF_3$ | H | 3-Cl | H | $CH_3$ | $CH_2CH_3$ | 1 | 1 | | |
| 341 | 2-$CH_3$<br>3-F<br>4-$CF(CF_3)CF_3$ | H | 3-Br | H | $CH_3$ | $CH_2CH_3$ | 1 | 1 | | |
| 342 | 2-$CH_3$<br>3-F<br>4-$CF(CF_3)CF_3$ | H | 3-$NO_2$ | H | $CH_3$ | $CH_2CH_3$ | 1 | 1 | | |
| 343 | 2-$CH_3$<br>3-F<br>4-$CF(CF_3)CF_3$ | H | 3-<br>$OSO_2CH_3$ | H | $CH_3$ | $CH_2CH_3$ | 1 | 1 | | |
| 344 | 2-$CH_3$<br>3-F<br>4-$CF(CF_3)CF_3$ | H | 3-$SO_2CH_3$ | H | $CH_3$ | $CH_2CH_3$ | 1 | 1 | | |
| 345 | 2-$CH_3$<br>3-F<br>4-$CF(CF_3)CF_3$ | H | 3-$SO_2CF_3$ | H | $CH_3$ | $CH_2CH_3$ | 1 | 1 | | |
| 346 | 2-$CH_3$<br>3-F<br>4-$CF(CF_3)CF_3$ | H | 3-$CF_3$ | H | $CH_3$ | $CH_2CH_3$ | 1 | 1 | | |
| 347 | 2-$CH_3$<br>3-Cl<br>4-$CF_2CF_3$ | H | 3-Cl | H | $CH_3$ | $CH_2CH_3$ | 1 | 2 | 3.14[a] | 91-92 |
| 348 | 2-$CH_3$<br>3-Cl<br>4-$CF_2CF_3$ | H | 3-Cl | H | $CH_3$ | $CH_2CH_3$ | 1 | 2 | | |
| 349 | 2-$CH_3$<br>3-Cl<br>4-$CF_2CF_3$ | H | 3-Br | H | $CH_3$ | $CH_2CH_3$ | 1 | 2 | | |
| 350 | 2-$CH_3$<br>3-Cl<br>4-$CF_2CF_3$ | H | 3-$NO_2$ | H | $CH_3$ | $CH_2CH_3$ | 1 | 2 | | |
| 351 | 2-$CH_3$<br>3-Cl<br>4-$CF_2CF_3$ | H | 3-<br>$OSO_2CH_3$ | H | $CH_3$ | $CH_2CH_3$ | 1 | 2 | | |
| 352 | 2-$CH_3$<br>3-Cl<br>4-$CF_2CF_3$ | H | 3-$SO_2CH_3$ | H | $CH_3$ | $CH_2CH_3$ | 1 | 2 | | |
| 353 | 2-$CH_3$<br>3-Cl<br>4-$CF_2CF_3$ | H | 3-$SO_2CF_3$ | H | $CH_3$ | $CH_2CH_3$ | 1 | 2 | | |
| 354 | 2-$CH_3$<br>3-Cl<br>4-$CF_2CF_3$ | H | 3-$CF_3$ | H | $CH_3$ | $CH_2CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 355 | 2-$CH_3$<br>3-F<br>4-$CF_2CF_3$ | H | 3-Cl | H | $CH_3$ | $CH_3$ | 1 | 0 | 3.94[b] | 190-193 |
| 356 | 2-$CH_3$<br>3-F<br>4-$CF_2CF_3$ | H | 3-1 | H | $CH_3$ | $CH_3$ | 1 | 0 | | |
| 357 | 2-$CH_3$<br>3-F<br>4-$CF_2CF_3$ | H | 3-Br | H | $CH_3$ | $CH_3$ | 1 | 0 | | |
| 358 | 2-$CH_3$<br>3-F<br>4-$CF_2CF_3$ | H | 3-$NO_2$ | H | $CH_3$ | $CH_3$ | 1 | 0 | | |
| 359 | 2-$CH_3$<br>3-F<br>4-$CF_2CF_3$ | H | 3-<br>$OSO_2CH_3$ | H | $CH_3$ | $CH_3$ | 1 | 0 | | |
| 360 | 2-$CH_3$<br>3-F<br>4-$CF_2CF_3$ | H | 3-$SO_2CH_3$ | H | $CH_3$ | $CH_3$ | 1 | 0 | | |
| 361 | 2-$CH_3$<br>3-F<br>4-$CF_2CF_3$ | H | 3-$SO_2CF_3$ | H | $CH_3$ | $CH_3$ | 1 | 0 | | |
| 362 | 2-$CH_3$<br>3-F<br>4-$CF_2CF_3$ | H | 3-$CF_3$ | H | $CH_3$ | $CH_3$ | 1 | 0 | | |
| 363 | 2-$CH_3$<br>3-F<br>4-$CF_2CF_3$ | H | 3-Cl | H | $CH_3$ | $CH_3$ | 1 | 2 | 2.82[a] | |
| 364 | 2-$CH_3$<br>3-F<br>4-$CF_2CF_3$ | H | 3-1 | H | $CH_3$ | $CH_3$ | 1 | 2 | | |
| 365 | 2-$CH_3$<br>3-F<br>4-$CF_2CF_3$ | H | 3-Br | H | $CH_3$ | $CH_3$ | 1 | 2 | | |
| 366 | 2-$CH_3$<br>3-F<br>4-$CF_2CF_3$ | H | 3-$NO_2$ | H | $CH_3$ | $CH_3$ | 1 | 2 | | |
| 367 | 2-$CH_3$<br>3-F<br>4-$CF_2CF_3$ | H | 3-<br>$OSO_2CH_3$ | H | $CH_3$ | $CH_3$ | 1 | 2 | | |
| 368 | 2-$CH_3$<br>3-F<br>4-$CF_2CF_3$ | H | 3-$SO_2CH_3$ | H | $CH_3$ | $CH_3$ | 1 | 2 | | |
| 369 | 2-$CH_3$<br>3-F<br>4-$CF_2CF_3$ | H | 3-$SO_2CF_3$ | H | $CH_3$ | $CH_3$ | 1 | 2 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 370 | 2-$CH_3$<br>3-F<br>4-$CF_2CF_3$ | H | 3-$CF_3$ | H | $CH_3$ | $CH_3$ | 1 | 2 | | |
| 371 | 2-$CH_3$<br>4-CF($CHF_2$)<br>$CF_3$ | H | 3-Cl | H | $CH_3$ | $CH_3$ | 1 | 2 | 2.61[a]<br>2.64[c] | 101-103 |
| 372 | 2-$CH_3$<br>3-F<br>4-$CF_2CF_3$ | H | 3-Cl | H | $CH_3$ | $CH_2CH_3$ | 1 | 2 | 3.01[a] | |
| 373 | 2-$CH_3$<br>3-F<br>4-$CF_2CF_3$ | H | 3-1 | H | $CH_3$ | $CH_2CH_3$ | 1 | 2 | | |
| 374 | 2-$CH_3$<br>3-F<br>4-$CF_2CF_3$ | H | 3-Br | H | $CH_3$ | $CH_2CH_3$ | 1 | 2 | | |
| 375 | 2-$CH_3$<br>3-F<br>4-$CF_2CF_3$ | H | 3-$NO_2$ | H | $CH_3$ | $CH_2CH_3$ | 1 | 2 | | |
| 376 | 2-$CH_3$<br>3-F<br>4-$CF_2CF_3$ | H | 3-<br>$OSO_2CH_3$ | H | $CH_3$ | $CH_2CH_3$ | 1 | 2 | | |
| 377 | 2-$CH_3$<br>3-F<br>4-$CF_2CF_3$ | H | 3-$SO_2CH_3$ | H | $CH_3$ | $CH_2CH_3$ | 1 | 2 | | |
| 378 | 2-$CH_3$<br>3-F<br>4-$CF_2CF_3$ | H | 3-$SO_2CF_3$ | H | $CH_3$ | $CH_2CH_3$ | 1 | 2 | | |
| 379 | 2-$CH_3$<br>3-F<br>4-$CF_2CF_3$ | H | 3-$CF_3$ | H | $CH_3$ | $CH_2CH_3$ | 1 | 2 | | |
| 380 | 2-$CH_3$<br>3-F<br>4-$CF_2CF_3$ | H | 3-Cl | H | $CH_3$ | $CH_2CH_3$ | 1 | 0 | 4.21[a] | 116-120 |
| 381 | 2-$CH_3$<br>3-F<br>4-$CF_2CF_3$ | H | 3-1 | H | $CH_3$ | $CH_2CH_3$ | 1 | 0 | | |
| 382 | 2-$CH_3$<br>3-F<br>4-$CF_2CF_3$ | H | 3-Br | H | $CH_3$ | $CH_2CH_3$ | 1 | 0 | | |
| 383 | 2-$CH_3$<br>3-F<br>4-$CF_2CF_3$ | H | 3-$NO_2$ | H | $CH_3$ | $CH_2CH_3$ | 1 | 0 | | |
| 384 | 2-$CH_3$<br>3-F<br>4-$CF_2CF_3$ | H | 3-<br>$OSO_2CH_3$ | H | $CH_3$ | $CH_2CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 385 | 2-$CH_3$ 3-F 4-$CF_2CF_3$ | H | 3-$SO_2CH_3$ | H | $CH_3$ | $CH_2CH_3$ | 1 | 0 | | |
| 386 | 2-$CH_3$ 3-F 4-$CF_2CF_3$ | H | 3-$SO_2CF_3$ | H | $CH_3$ | $CH_2CH_3$ | 1 | 0 | | |
| 387 | 2-$CH_3$ 3-F 4-$CF_2CF_3$ | H | 3-$CF_3$ | H | $CH_3$ | $CH_2CH_3$ | 1 | 0 | | |
| 388 | 2-$CH_3$ 3-F 4-$CF_2CF_3$ | H | 3-Br | H | $CH_3$ | $CH_3$ | 1 | 0 | 3.93[a] | 150-155 |
| 389 | 2-$CH_3$ 3-F 4-$CF_2CF_3$ | H | 3-Br | H | $CH_3$ | $CH_3$ | 1 | 2 | 2.86[a] | 88-94 |
| 390 | 2-$CH_3$ 4-$CF(CF_3)CF_3$ | H | 3-Cl | H | $CH_3$ | $CH_3$ | 1 | 0 | | |
| 391 | 2-$CH_3$ 4-$CF(CF_3)CF_3$ | H | 3-1 | H | $CH_3$ | $CH_3$ | 1 | 0 | | |
| 392 | 2-$CH_3$ 4-$CF(CF_3)CF_3$ | H | 3-Br | H | $CH_3$ | $CH_3$ | 1 | 0 | | |
| 393 | 2-$CH_3$ 4-$CF(CF_3)CF_3$ | H | 3-$NO_2$ | H | $CH_3$ | $CH_3$ | 1 | 0 | | |
| 394 | 2-$CH_3$ 4-$CF(CF_3)CF_3$ | H | 3-$OSO_2CH_3$ | H | $CH_3$ | $CH_3$ | 1 | 0 | | |
| 395 | 2-$CH_3$ 4-$CF(CF_3)CF_3$ | H | 3-$SO_2CH_3$ | H | $CH_3$ | $CH_3$ | 1 | 0 | | |
| 396 | 2-$CH_3$ 4-$CF(CF_3)CF_3$ | H | 3-$SO_2CF_3$ | H | $CH_3$ | $CH_3$ | 1 | 0 | | |
| 397 | 2-$CH_3$ 4-$CF(CF_3)CF_3$ | H | 3-$CF_3$ | H | $CH_3$ | $CH_3$ | 1 | 0 | | |
| 398 | 2-$CH_3$ 3-F 4-$CF(CF_3)CF_3$ | H | 3-Br | H | $CH_3$ | $CH_3$ | 1 | 0 | 4.31[a] | |
| 399 | 2-$CH_3$ 3-F 4-$CF(CF_3)CF_3$ | H | 3-Cl | H | $CH_3$ | $CH_3$ | 1 | 0 | | |
| 400 | 2-$CH_3$ 3-F 4-$CF(CF_3)CF_3$ | H | 3-1 | H | $CH_3$ | $CH_3$ | 1 | 0 | | |
| 401 | 2-$CH_3$ 3-F 4-$CF(CF_3)CF_3$ | H | 3-$NO_2$ | H | $CH_3$ | $CH_3$ | 1 | 0 | | |
| 402 | 2-$CH_3$ 3-F 4-$CF(CF_3)CF_3$ | H | 3-$OSO_2CH_3$ | H | $CH_3$ | $CH_3$ | 1 | 0 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 403 | 2-$CH_3$ 3-F 4-$CF(CF_3)CF_3$ | H | 3-$SO_2CH_3$ | H | $CH_3$ | $CH_3$ | 1 | 0 | | |
| 404 | 2-$CH_3$ 3-F 4-$CF(CF_3)CF_3$ | H | 3-$SO_2CF_3$ | H | $CH_3$ | $CH_3$ | 1 | 0 | | |
| 405 | 2-$CH_3$ 3-F 4-$CF(CF_3)CF_3$ | H | 3-$CF_3$ | H | $CH_3$ | $CH_3$ | 1 | 0 | | |
| 406 | 2-$CH_3$ 3-F 4-$CH(CF_3)CF_3$ | H | 3-Cl | H | $CH_3$ | $CH_3$ | 1 | 1 | 2.5[a] 2.41[c] | 116-118 |
| 407 | 2-$CH_3$ 3-F 4-$CH(CF_3)$ $CF_3$ | H | 3-1 | H | $CH_3$ | $CH_3$ | 1 | 1 | | |
| 408 | 2-$CH_3$ 3-F 4-$CH(CF_3)$ $CF_3$ | H | 3-Br | H | $CH_3$ | $CH_3$ | 1 | 1 | | |
| 409 | 2-$CH_3$ 3-F 4-$CH(CF_3)$ $CF_3$ | H | 3-$NO_2$ | H | $CH_3$ | $CH_3$ | 1 | 1 | | |
| 410 | 2-$CH_3$ 3-F 4-$CH(CF_3)$ $CF_3$ | H | 3-$OSO_2CH_3$ | H | $CH_3$ | $CH_3$ | 1 | 1 | | |
| 411 | 2-$CH_3$ 3-F 4-$CH(CF_3)$ $CF_3$ | H | 3-$SO_2CH_3$ | H | $CH_3$ | $CH_3$ | 1 | 1 | | |
| 412 | 2-$CH_3$ 3-F 4-$CH(CF_3)$ $CF_3$ | H | 3-$SO_2CF_3$ | H | $CH_3$ | $CH_3$ | 1 | 1 | | |
| 413 | 2-$CH_3$ 3-F 4-$CH(CF_3)$ $CF_3$ | H | 3-$CF_3$ | H | $CH_3$ | $CH_3$ | 1 | 1 | | |
| 414 | 2-$CH_3$ 4-$CH(CF_3)$ $CF_3$ 5-F | H | 3-Cl | H | $CH_3$ | $CH_3$ | 1 | 1 | 2.58[a] 2.51[c] | 130-135 |
| 415 | 2-$CH_3$ 4-$CH(CF_3)$ $CF_3$ 5-F | H | 3-1 | H | $CH_3$ | $CH_3$ | 1 | 1 | | |

(continued)

| No. | $Y_n$ | $R^2$ | $X_m$ | $R^1$ | $R^{3*}$ | A | p | q | logP | melting point [°C] |
|---|---|---|---|---|---|---|---|---|---|---|
| 416 | 2-$CH_3$<br>4-$CH(CF_3)$<br>$CF_3$<br>5-F | H | 3-Br | H | $CH_3$ | $CH_3$ | 1 | 1 | | |
| 417 | 2-$CH_3$<br>4-$CH(CF_3)$<br>$CF_3$<br>5-F | H | 3-$NO_2$ | H | $CH_3$ | $CH_3$ | 1 | 1 | | |
| 418 | 2-$CH_3$<br>4-$CH(CF_3)$<br>$CF_3$<br>5-F | H | 3-<br>$OSO_2CH_3$ | H | $CH_3$ | $CH_3$ | 1 | 1 | | |
| 419 | 2-$CH_3$<br>4-$CH(CF_3)$<br>$CF_3$<br>5-F | H | 3-$SO_2CH_3$ | H | $CH_3$ | $CH_3$ | 1 | 1 | | |
| 420 | 2-$CH_3$<br>4-$CH(CF_3)$<br>$CF_3$<br>5-F | H | 3-$SO_2CF_3$ | H | $CH_3$ | $CH_3$ | 1 | 1 | | |
| 421 | 2-$CH_3$<br>4-$CH(CF_3)$<br>$CF_3$<br>5-F | H | 3-$CF_3$ | H | $CH_3$ | $CH_3$ | 1 | 1 | | |
| 422 | 2-$CH_3$<br>4-$CH(CF_3)$<br>$CF_3$<br>5-F | H | 3-Br | H | $CH_3$ | $CH_3$ | 1 | 1 | 2.58[a]<br>2.51[c] | |
| 423 | 2-$CH_3$<br>3-F<br>4-$CF(CF_3)CF_3$ | H | 3-1 | H | $CH_3$ | $CH_3$ | 1 | 0 | 4,41[a] | |
| 424 | 2-Br<br>4-$CF(CHF_2)$<br>$CH_3$ | H | 3-Br | H | $CH_3$ | $CH_2CH_3$ | 1 | 2 | 3,06[a] | 75-79°C |
| 425 | 2-$CH_3$<br>4-$CF(CF_3)CF_3$ | H | 3-1 | H | $CH_3$ | $CH_3$ | 1 | 2 | | |

[a] 0.1% aqueous phosphoric acid and acetonitrile at pH 2.3; linear gradient from 10 to 95 % (v/v) acetonitrile.
[b] 0.1% aqueous formic acid and acetonitrile at pH 2.7; linear gradient from 10 % to 95 % (v/v) acetonitrile.
[c] 0.001 molar aqueous $NH_4HCO_3$ solution and acetonitrile at pH 7.8; linear gradient from 10 % to 95 % acetonitrile (v/v).

Determination of the logP values:

The logP values presented herein were determined in accordance with EEC Directive 79/831 Annex V.A8 by HPLC (High Performance Liquid Chromatography) on a reversed-phase column (C 18) at a temperature of 40 °C, using the following method:

Calibration was carried out using unbranched alkan-2-ones (comprising 3 to 16 carbon atoms) with known logP values (determination of the logP values by the retention times using linear interpolation between two successive alkanones). The lambda max values were determined in the maxima of the chromatographic signals using the UV

spectra from 190 nm to 400 nm.

The present invention will be further described in the following examples. However. these examples are not intended to limit the scope of the present application.

Examples

When not mentioned otherwise the tested compounds or compositions were administered in a suitable formulation.

Example No.1

**Boophilus microplus - test**

Solvent: dimethylsulfoxide

To produce a suitable preparation of active compound, 1 part by weight of active compound is mixed with the stated amount of solvent, and the concentrate is diluted with containing solvent to the desired concentration.

Adult ticks (Boophilus microplus) are treated by injected with compound solution of the desired concentration into the abdomen. Ticks are transferred into replica plates and incubated in a climate chamber for seven days. Eggs which are not clearly fertile or infertile are further incubated for another 35 days until larvae hatch from the untreated controls.

After the specified period of time, the laying of fertile eggs in % is determined. 100 % means that all eggs have been fertile; 0 % means that none of the eggs have been fertile.

In this test for example, the following compounds from the preparation examples showed good activity of ≥ 80 % at application rate of 20 μg/animal:

Compounds Nos.: 1, 24, 48, 56, 80, 129, 331, 339, 372, 355, 347, 363, 372, 388, 389, 398, 105, 138, 146, 423, 170, 267, 424, 323, 154, 218, 202, 210, 227, 235, 243, 219, 251, 291, 299, 315, 371, 390, 406, 414, 422.

Example No. 2

**Musca domestica - test**

Solvent: dimethyl sulfoxide

To produce a suitable preparation of active compound, 10 mg of active compound are dissolved in 0.5 ml solvent, and the concentrate is diluted with water to the desired concentration.

Prior to the assay, a piece or kitchen sponge is soaked with a mixture of sugar and compound solution and placed into a container. 10 adults (Musca domestica) are placed into the container and closed with a perforated lid.

In this test for example, the following compounds from the preparation examples showed good activity of ≥ 80 % at application rate of 20ppm:

Example Nos.: 424, 372.

After the specified period of time, mortality in % is determined. 100 % means that all the flies have been killed; 0 % means that none of the flies have been killed.

In this test for example, the following compounds from the preparation examples showed good activity of ≥ 80 % at application rate of 100ppm:

Compounds Nos.: 24, 48, 56, 80, 23, 363, 398, 1, 130, 162, 423, 178, 424, 323, 218, 210, 227, 235, 243, 219, 251, 259, 275, 283, 291, 299, 307, 315, 371, 390, 406, 414.

Example No. 3

**Lucilia cuprina - test**

Solvent: dimethyl sulfoxide

To produce a suitable preparation of active compound, 10 mg of active compound are dissolved in 0.5 ml solvent, and the concentrate is diluted with water to the desired concentration.

Approximately 20 -30 (Lucilia cuprina larvae) are transferred into a test tube containing $1cm^3$ of minced horse meat and 0.5 ml aqueous dilution of test compound.

After the specified period of time, mortality in % is determined. 100 % means that all the larvae have been killed; 0 % means that none of the larvae have been killed.

In this test for example, the following compounds from the preparation examples showed good activity of ≥ 80 % at application rate of 100ppm:

Compounds Nos.: 1, 24, 48, 56, 80, 23, 129, 331, 339, 372, 355, 347, 363, 372, 388, 389, 398, 105, 145, 130, 138, 146, 162, 423, 170, 178, 186, 267, 424, 323, 154, 218, 202, 210, 227, 235, 243, 251, 259, 275, 283, 291, 299, 307, 315, 371, 390, 406, 414, 422.

Formulation Example No. 1

Preparation of 20% soluble concentrate (SC) formulation comprising mixing

20 % (w/w) of compound no. 80 with
0.43 % (w/w) of dioctyl sodium sulfosuccinate,
0.43 % (w/w) of Veegum™,
0.26 % (w/w) of Xanthane,
8.57 % (w/w) of 1,2-propylene glycole,
0.17 % (w/w) of sodium propionate,
0.17 % (w/w) sodium benzoate, and
1.0 % (w/w) of Simethicone Emus. USP 30%, and Aqua demin. up to a volume of 2500 ml.

Formulation Example No. 2

Ear tag formulation based on compound no. 80 comprising mixing

71 g polyvinyl chloride,
1.0 g titanium dioxide,
20.0 g of compound no. 80,
0.3 g of the colorant D&C Violet™, and
20.0 g piperonyl butoxide,

and subjecting the mixture to injection moulding. The resulting ear tag is suitable for controlling parasites when fixed on the ears of cattle.

Formulation Example No. 3

Preparation of an emulsifiable concentrate (EC) formulation in a known manner comprising mixing

50.0 g of compound no. 80,
200.0 g the non ionic emulgator Emulsogen™ EL, purchased from Sigma Corp. Germany, and
855.0 g benzyl benzoate and
aqua demin. to yield a total volume of 1000 ml.

Formulation Example 4

Preparation of a spot-on formulation comprising

100.0 g of compound no. 80,
664.0 g Diethylene glycol monomethylether,
332.0 g Propylene carbonate and
1.0 g BHT (butylhydroxy toluene)
aqua demin. to yield a total volume of 1000 ml.

Example No. 4

**In-vivo testing against ticks on cattle (stall test)**

Cattle are infested with larvae of the one-host tropical cattle tick Rhipicephalus (Boophilus) microplus every second day for three weeks. On day 0 an aqueous dilution is prepared from the formulation example 1 and the resulting solution is transferred to a motor driven high pressure spray device. Animals in treatment groups are sprayed to saturation with 12 to 16 litres of the solution at a pressure of at least 5 bar. Tick development in treatment groups are compared to untreated controls for four weeks. Parameters, namely number of ticks dropping from the cattle, tick weight, tick mortality, egg weight and larval hatch rates area are assessed. Main efficacy criterion, the reproductive efficacy (RE), is calculated according to the following equation:

$$RE = Me/Mf * N/S *H/4,$$

Me = egg weight,

Mf = tick weight,

N = number of ticks incubated,

S = number of ticks survived,

H = Larval hatch rate on a scale from 1 (low) to 4 (normal).

The efficacy E is calculated according to the following equation:

$$E = (1 - (RET / REC)) * 100$$

RE = reproductive efficacy, T = treated group, C = untreated control group

Formulation example 1 showed an averaged efficacy against ticks of more than 25% at a dosage of 175 ppm over a period of three weeks.

Example No. 5

**In-vivo test against biting flies on cattle (stall test)**

On day 0 an aqueous dilution is prepared from the formulation example 1 and the resulting solution is transferred to a motor driven high pressure spray device. Animals in treatment groups are sprayed to saturation with 12 to 16 litres of the solution at a pressure of at least 5 bar. In a contact test design the efficacy of the treatment against buffalo flies (Haematobia irritans exigua) is compared to untreated animals. Plastic beakers with gauze net lids containing approximately 20 unfed female buffalo flies are put in contact with the skin on the backline of the animals for three minutes. After contact, flies are kept for 48 hrs at 25°C and 85% relative humidity, and fly mortality is assessed after 3, 24 and 48 hrs. Efficacy is calculated using the following equation:

$$E = \left(1 - \frac{\text{Number of dead flies after contact with treated animals}}{\text{Number of dead flies after contact with untreated animals}}\right) * 100$$

Formulation example 1 showed an averaged efficacy against buffalo flies of more than 30% at a dosage of 175 ppm.

Example No. 6

**In-vivo test against biting lice on sheep (Field test)**

Sheep infested with biting lice (Bovicola ovis) are sheared two weeks prior to treatment. On day 0 sheep are completely immersed in 200 litres of an aqueous dilution of the formulation example 1 (175 ppm). For four months lice infestation of treatment groups are compared to an untreated control group. At each efficacy assessment, the number of lice and lice developmental stages is counted in 10 different wool partings on each animal, as well as the number of adults, nymphs, larvae and eggs. To correct for the different pre-treatment infestation rates in each group efficacy calculation is performed using the formula of Henderson and Tilton:

$$E = 100 \text{ x } (1 - (Ba/Ka \text{ x } Kb/Bb))$$

Ba = total lice number after treatment on assessment day;

Bb = lice number of treatment group before treatment;

Kb = lice number of untreated control group before treatment of treatment group;

Ka = lice number in untreated control group on assessment day.

Formulation example 1 showed an averaged efficacy against biting lice of more than 90% at a dosage of 175 ppm for at least 4 month.

Example No. 7

**In-vivo test against biting lice on Angora goats (Field test)**

Angora goats infested with biting lice (Bovicola limbata) are assigned to three groups prior to treatment. On day 0 and day +8 goats are completely immersed in 350 litres of an aqueous dilution of the formulation example 1 (175 ppm). For four months lice infestation of treatment groups are compared to an untreated control group. At each efficacy assessment, the number of lice and lice developmental stages is counted in 10 different wool partings on each animal, as well as the number of adults, nymphs, larvae and eggs. To correct for the different pre-treatment infestation rates in each group efficacy calculation is performed using the formula of Henderson and Tilton:

$$E = 100 \text{ x } (1 - (Ba/Ka \text{ x } Kb/Bb))$$

Ba = total lice number after treatment on assessment day;

Bb = lice number of treatment group before treatment;

Kb = lice number of untreated control group before treatment of treatment group;

Ka = lice number in untreated control group on assessment day.

Formulation example 1 showed an averaged efficacy against biting lice of more than 90% at a dosage of 175 ppm for at least 4 month.

Example No. 8

**In-vivo test against sheep blowfly on sheep (Field test)**

150 L of an aqueous dilution of the formulation example 1 (175 ppm) is prepared on day 0 and transferred to the container of a high pressure spray device. Sheep from the treatment groups are jetted to saturation of the wool. Wool has been allowed to grow on the sheeps for about 12 weeks after last shearing. On week 4, 5, 6, 7, 9, 11 and 13 after treatment first stage larvae of the sheep blowfly Lucilia cuprina are placed on designated sites in the wool, and larval development after 2 days is estimated. Subsequently, surviving larvae are killed by acetone treatment. Survival rate and development times of larvae on treated sheep are compared to that of untreated animals and to a positive control group treated with a standard product Vetrazin. Efficacy is calculated as follows:

$$E = \left(1 - \frac{(\text{DS untreated control} - \text{DS investigational veterinary product treatment}}{\text{DS untreated control} - \text{DS standard product treatment})}\right) * 100$$

DS = average developmental stage of larvae after 48 h.

Formulation example 1 (175 ppm) showed a 50% average inhibitory effect on fly development.

**Claims**

1. Veterinary pharmaceutical composition comprising at least one optically active phthalamide compound of formula (I), or a salt thereof

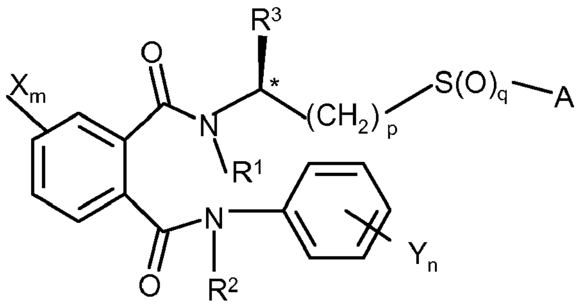

(I)

wherein

$R^1$ and $R^2$ independently of each other represents hydrogen, ($C_1$-$C_6$)-alkyl, ($C_1$-$C_6$)-alkyl which is substituted with at least one substituent selected from the group consisting of halogen, cyano, ($C_1$-$C_6$)-alkoxy, ($C_1$-$C_6$)-alkylthio, mono-($C_1$-$C_6$)-alkylamino, and di-($C_1$-$C_6$)-alkylamino in which the alkyl groups may be same or different, or ($C_1$-$C_6$)-alkoxycarbonyl;
$R^3$ represents ($C_1$-$C_6$)-alkyl;
A represents hydrogen, ($C_1$-$C_6$)-alkyl, halo($C_1$-$C_6$)-alkyl, ($C_3$-$C_6$)-alkeny, halo($C_3$-$C_6$)-alkenyl, ($C_3$-$C_6$)-alkynyl, halo($C_3$-$C_6$)-alkynyl, ($C_1$-$C_6$)-alkoxy($C_1$-$C_6$)-alkyl, halo($C_1$-$C_6$)-alkoxy($C_1$-$C_6$)alkyl, ($C_1$-$C_6$)-alkylthio ($C_1$-$C_6$)-alkyl, halo($C_1$-$C_6$)-alkylthio($C_1$-$C_6$)-alkyl, ($C_1$-$C_6$)-alkylsulfinyl($C_1$-$C_6$)-alkyl, halo($C_1$-$C_6$)alkylsulfinyl ($C_1$-$C_6$)-alkyl, ($C_1$-$C_6$)-alkylsulfonyl($C_1$-$C_6$)-alkyl, halo($C_1$-$C_6$)alkylsulfonyl($C_1$-$C_6$)-alkyl, tri-($C_1$-$C_6$)-alkylsilyl, ($C_1$-$C_6$)-alkylcarbonyl, mono-($C_1$-$C_6$)-alkyl-carbamoyl, di-($C_1$-$C_6$)-alkylcarbamoyl in which the alkyl groups may be same or different, phenoxy($C_1$-$C_6$)-alkyl, substituted phenoxy ($C_1$-$C_6$)-alkyl having one or more substituents that may be same or different and are selected from the group consisting of halogen, ($C_1$-$C_6$)-alkyl, halo

$(C_1-C_6)$-alkyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di-(halo$(C_1-C_6)$-alkyl)amino in which the alkyl groups may be same or different, phenyl$(C_1-C_6)$-alkyl, or substituted phenyl$(C_1-C_6)$-alkyl wherein the ring is substituted with, one or more substituents that may be same or different and are selected from the group consisting of halogen, $(C_1-C_6)$-alkyl, halo$(C_1-C_6)$-alkyl, $(C_1-C_6)$-alkoxy, halo $(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di-(halo$(C_1-C_6)$-alkyl)amino in which the alkyl groups may be same or different;

p represents 0, 1, 2, 3 or 4;

q represents 0, 1, or 2;

X may be same or different and represents halogen, cyano, amino, nitro, $(C_1-C_6)$-alkyl, halo$(C_1-C_6)$-alkyl, $(C_2-C_6)$-alkenyl, halo$(C_2-C_6)$-alkenyl, $(C_2-C_6)$-alkynyl, halo$(C_2-C_6)$-alkynyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, $(C_1-C_6)$-alkylsulfonyloxy, halo$(C_1-C_6)$-alkylsulfonyl, mono-$(C_1-C_6)$-alkylamino, or di-$(C_1-C_6)$-alkylamino in which the alkyl groups may be same or different; and

m represents 0, 1, 2, 3 or 4; and

Y may be same or different and represents hydrogen, halogen, cyano, amino, nitro, $(C_1-C_6)$-alkyl, halo $(C_1-C_6)$-alkyl, $(C_2-C_6)$-alkenyl, halo$(C_2-C_6)$-alkenyl, $(C_2-C_6)$-alkynyl, halo$(C_2-C_6)$-alkynyl, $(C_3-C_6)$-cycloalkyl, halo$(C_3-C_6)$-cycloalkyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino, in which the alkyl groups may be same or different, tri-$(C_1-C_6)$-alkylsilyl in which the alkyl groups may be same or different, phenyl, substituted phenyl having one or more substituents that may be same or different and are selected from the group consisting of halogen, $(C_1-C_6)$-alkyl, halo$(C_1-C_6)$-alkyl, $(C_1-C_6)$-alkoxy group, halo$(C_1-C_6)$-alkoxy, $(C_1-C_6)$-alkylthio, halo $(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di(halo $(C_1-C_6)$-alkyl)amino in which the alkyl groups may be same or different, heterocyclic, or a substituted heterocyclic having one or more substituents that may be same or different and are selected from the group consisting of halogen, $(C_1-C_6)$-alkyl, halo$(C_1-C_6)$-alkyl, $(C_1-C_6)$-alkoxy, halo $(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo$(C_1-C_6)$-alkylsulfonyl, mono-$(C_1-C_6)$-alkylamino, di-$(C_1-C_6)$-alkylamino in which the alkyl groups may be same or different, mono-(halo$(C_1-C_6)$-alkyl)amino, and di-(halo$(C_1-C_6)$-alkyl)amino in which the alkyl groups may be same or different; and

n represents 1, 2, 3, 4, or 5;

for preventing infection with diseases transmitted through parasites.

**2.** Composition according to claim 1, wherein

$R^1$ and $R^2$ independently of each other represents hydrogen or $(C_1-C_6)$-alkyl;

$R^3$ represents methyl or ethyl;

A represents $(C_1-C_6)$-alkyl, $(C_3-C_6)$-alkenyl, $(C_3-C_6)$-alkynyl, $(C_1-C_6)$-alkoxy$(C_1-C_6)$-alkyl, $(C_1-C_6)$-alkylthio $(C_1-C_6)$-alkyl, $(C_1-C_6)$-alkylsulfinyl$(C_1-C_6)$-alkyl, $(C_1-C_6)$-alkylsulfonyl$(C_1-C_6)$-alkyl, or a mono-$(C_1-C_6)$-alkylcarbamoyl group;

p represents 0, 1, 2, 3 or 4;

q represents 0, 1, or 2;

X may be same or different and represents halogen, nitro, halo$(C_1-C_6)$-alkyl, halo$(C_1-C_6)$-alkoxy, $(C_1-C_6)$-alkylthio, $(C_1-C_6)$-alkylsulfinyl, $(C_1-C_6)$-alkylsulfonyl, $(C_1-C_6)$-alkylsulfonyloxy;

m represents 0, 1, or 2;

Y may be same or different and represents halogen, cyano, $(C_1-C_6)$-alkyl, halo$(C_1-C_6)$-alkyl, $(C_2-C_6)$-alkenyl, halo$(C_2-C_6)$-alkenyl, $(C_2-C_6)$-alkynyl, halo$(C_2-C_6)$-alkynyl, $(C_1-C_6)$-alkoxy, halo$(C_1-C_6)$-alkoxy, $(C_1-C_6)$-alkylthio, halo$(C_1-C_6)$-alkylthio, $(C_1-C_6)$-alkylsulfinyl, halo$(C_1-C_6)$-alkylsulfinyl, $(C_1-C_6)$-alkylsulfonyl, halo $(C_1-C_6)$-alkylsulfonyl, mono-(halo$(C_1-C_6)$-alkyl)amino, or di-(halo$(C_1-C_6)$-alkyl)amino in which the alkyl groups may be same or different;

and

n represents 0, 1, 2, 3, or 4.

**3.** Composition according to claim 1, wherein

$R^1$ and $R^2$ independently of each other represents hydrogen or ($C_1$-$C_6$)-alkyl;
$R^3$ represents methyl;
A represents ($C_1$-$C_6$)-alkyl, ($C_3$-$C_6$)-alkenyl, ($C_3$-$C_6$)-alkynyl, ($C_1$-$C_6$)-alkoxy($C_1$-$C_6$)-alkyl, ($C_1$-$C_6$)-alkylthio ($C_1$-$C_6$)-alkyl, ($C_1$-$C_6$)-alkylsulfinyl($C_1$-$C_6$)-alkyl, or ($C_1$-$C_6$)-alkylsulfonyl($C_1$-$C_6$)-alkyl;
p represents 1 or 2;
q represents 0, 1 or 2;
X may be same or different and represents halogen, nitro, halo($C_1$-$C_6$)-alkyl, or halo ($C_1$-$C_6$)-alkoxy, ($C_1$-$C_6$)-alkylthio, ($C_1$-$C_6$)-alkylsulfinyl, ($C_1$-$C_6$)-alkylsulfonyl, ($C_1$-$C_6$)-alkylsulfonyloxy;
m represents 1, or 2;

and

Y may be same or different and represents halogen, cyano, ($C_1$-$C_6$)-alkyl, halo($C_1$-$C_6$)-alkyl, ($C_2$-$C_6$)-alkenyl, halo($C_2$-$C_6$)-alkenyl, ($C_2$-$C_6$)-alkynyl, halo($C_2$-$C_6$)-alkynyl, ($C_1$-$C_6$)-alkoxy, halo($C_1$-$C_6$)-alkoxy, ($C_1$-$C_6$)-alkylthio, halo($C_1$-$C_6$)-alkylthio, ($C_1$-$C_6$)-alkylsulfinyl, halo($C_1$-$C_6$)-alkylsulfinyl, ($C_1$-$C_6$)-alkylsulfonyl, or halo ($C_1$-$C_6$)-alkylsulfonyl, and
n represents 1, 2, or 3.

**4.** Composition according to claim 1, wherein the optically active phthalamide compound is selected from the group consisting of (S)-3-iodo-$N^1$-{2-methyl-4-[1,2,2,2-tetrafluoro-1-(trifluoromethyl)ethyl]phenyl}-$N^2$-(1-methyl-2-methylsulfonylethyl)phthalamide, (S)-3-chloro-N'-{2-methyl-4-[1,2,2,2-tetrafluoro-1-(trifluoromethyl)ethyl]phenyl}-$N^2$-(1-methyl-2-methylsulfonylethyl)phthalamide, (S)-3-bromo-$N^1$-{2-methyl-4-[1,2,2,2-tetrafluoro-1-(trifluoromethyl) ethyl]phenyl}-$N^2$-(1-methyl-2-methylsulfonylethyl)phthalamide, and (S)-3-iodo-$N^1$-{2-methyl-4-[1,2,2,2-tetrafluoro-1-(trifluoromethyl)ethyl]phenyl}-$N^2$-(1-methyl-2-methylsulfinylethyl) phthalamide, or a salt thereof.

**5.** Use of the composition as claimed in any one of claims 1 to 4 for controlling animal parasites.

**6.** Use of the composition as claimed in any one of claims 1 to 4 for the preparation of a veterinary pharmaceutical for controlling animal parasites.

**7.** Use of the composition as claimed in any one of claims 1 to 4, for controlling arthropods.

**8.** Method for preventing infection with diseases transmitted through parasites **characterized in that** the composition as defined in any one of claims 1 to 4 is externally applied.

European Patent Office

# PARTIAL EUROPEAN SEARCH REPORT

which under Rule 63 of the European Patent Convention shall be considered, for the purposes of subsequent proceedings, as the European search report

Application Number
EP 07 11 4034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/024412 A (BAYER CROPSCIENCE AG [DE]; FISCHER RUEDIGER [DE]; FUNKE CHRISTIAN [DE]) 9 March 2006 (2006-03-09)<br>* page 17 - page 18 *<br>* page 27 * | 1-3,5-8 | INV.<br>A61K31/166<br>A61P33/00 |
| Y | * page 29 - page 31; table 1 *<br>* page 38 - page 39; table C *<br>* page 41 - page 42; table D *<br>----- | 1-8 | |
| X | WO 2006/133823 A (BAYER CROPSCIENCE AG [DE]; WADA KATSUAKI [JP]; MURATA TETSUYA [JP]; SH) 21 December 2006 (2006-12-21)<br>* page 43, line 23 - page 44, line 6 *<br>* page 51 - page 65; tables 1-8 *<br>----- | 1,5-8 | |
| X,E | WO 2007/101539 A (BAYER CROPSCIENCE AG [DE]; FISCHER RUEDIGER [DE]; FUNKE CHRISTIAN [DE])<br>13 September 2007 (2007-09-13)<br>* page 1 - page 2 *<br>* page 22, line 8 - line 28 *<br>* claims 1-11 *<br>-----<br>-/-- | 1-8 | TECHNICAL FIELDS SEARCHED (IPC)<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 4 February 2008 | Damiani, Federica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04E07)

European Patent Office

# PARTIAL EUROPEAN SEARCH REPORT

Application Number
EP 07 11 4034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 782 689 A (NIHON NOHYAKU CO LTD [JP]) 9 May 2007 (2007-05-09)<br>* page 11, line 1 - line 2 *<br>* page 18 - page 27; tables 1-3 *<br>----- | 5 | |
| Y | WO 2006/008108 A (BAYER CROPSCIENCE AG [DE]; FISCHER REINER [DE]; FISCHER RUEDIGER [DE];)<br>26 January 2006 (2006-01-26)<br>* page 30; table 4 *<br>* page 47 *<br>----- | 1-8 | |

TECHNICAL FIELDS SEARCHED (IPC)

2

EPO FORM 1503 03.82 (P04C10)

European Patent Office

**INCOMPLETE SEARCH SHEET C**

Application Number
EP 07 11 4034

Although claims 5, 7, 8 are directed to a method of treatment of the human/animal body (Article 53(c) EPC), the search has been carried out and based on the alleged effects of the compound/composition.

-----

## ANNEX TO THE EUROPEAN SEARCH REPORT ON EUROPEAN PATENT APPLICATION NO. EP 07 11 4034

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2008

| Patent document cited in search report | | Publication date | Patent family member(s) | | | Publication date |
|---|---|---|---|---|---|---|
| WO 2006024412 | A | 09-03-2006 | AU | 2005279429 | A1 | 09-03-2006 |
| | | | CN | 101048370 | A | 03-10-2007 |
| | | | KR | 20070054700 | A | 29-05-2007 |
| WO 2006133823 | A | 21-12-2006 | AR | 054134 | A1 | 06-06-2007 |
| | | | AU | 2006257416 | A1 | 21-12-2006 |
| | | | JP | 2006347936 | A | 28-12-2006 |
| WO 2007101539 | A | 13-09-2007 | DE | 102006010205 | A1 | 13-09-2007 |
| EP 1782689 | A | 09-05-2007 | AR | 050707 | A1 | 15-11-2006 |
| | | | AU | 2005275886 | A1 | 02-03-2006 |
| | | | CA | 2576325 | A1 | 02-03-2006 |
| | | | CN | 101006049 | A | 25-07-2007 |
| | | | WO | 2006022225 | A1 | 02-03-2006 |
| | | | KR | 20070046135 | A | 02-05-2007 |
| WO 2006008108 | A | 26-01-2006 | AU | 2005263567 | A1 | 26-01-2006 |
| | | | CA | 2574205 | A1 | 26-01-2006 |
| | | | CN | 1988804 | A | 27-06-2007 |
| | | | DE | 102004035134 | A1 | 16-02-2006 |
| | | | EP | 1771072 | A2 | 11-04-2007 |
| | | | KR | 20070039146 | A | 11-04-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1006107 A1
- EP 1782689 A2


### Non-patent literature cited in the description


- **TOHNISHI, M.** *J. Pestic. Sci.,* 2005, vol. 30 (4), 354-360